(19) Europäisches Patentamt
European Patent Office
Office européen des brevets

(11) Veröffentlichungsnummer: **0 133 247**
**B1**

(12) # EUROPÄISCHE PATENTSCHRIFT

(45) Veröffentlichungstag der Patentschrift: **13.12.89**

(51) Int. Cl.[4]: **C 07 D207/34**, C 07 D401/06, C 07 D403/06, C 07 D413/06, A 61 K 31/40

(21) Anmeldenummer: **84108547.5**

(22) Anmeldetag: **19.07.84**

(54) **N-Aminomethyl-3-phenyl-4-cyanopyrrol-Derivate, deren Herstellung und Verwendung als Mikrobizide.**

(30) Priorität: **22.07.83 CH 4031/83**

(43) Veröffentlichungstag der Anmeldung: **20.02.85 Patentblatt 85/08**

(45) Bekanntmachung des Hinweises auf die Patenterteilung: **13.12.89 Patentblatt 89/50**

(84) Benannte Vertragsstaaten: **AT BE CH DE FR GB IT LI LU NL SE**

(56) Entgegenhaltungen:
**DE-A- 2 927 480**
**GB-A- 2 013 187**
**TETRAHEDRON LETTERS, Nr. 52, Dezember 1972, Seiten 5337-5340, Pergamon Press, Oxford, GB; A.M. VAN LEUSEN et al.: "A new and simple synthesis of the pyrrole ring system from Michael acceptors and tosylmethylisocyanides"**

(73) Patentinhaber: **CIBA-GEIGY AG**
**Klybeckstrasse 141**
**CH-4002 Basel (CH)**

(72) Erfinder: **Nyfeler, Robert, Dr.**
**Bärenfelserstrasse 8**
**CH-4057 Basel (CH)**

(74) Vertreter: **Zumstein, Fritz sen., Dr. et al**
**Bräuhausstrasse 4**
**D-8000 München 2 (DE)**

**EP 0 133 247 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

## Beschreibung

Die vorliegende Erfindung betrifft neue N-Aminomethyl-3-phenyl-4-cyanopyrrol-derivate der nachstehenden Formel I, deren Herstellung, sowie mikrobizide Mittel, die als Wirkstoff mindestens eine der Titelverbindungen enthalten. Die Erfindung betrifft ferner die Herstellung der genannten Mittel und die Verwendung der neuen Wirkstoffe und Mittel zur Bekämpfung schädlicher Mikroorganismen, insbesondere pflanzenschädigender Pilze.

Bei den erfindungsgemässen Verbindungen handelt es sich um solche der Formel I

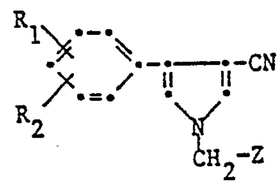

(I)

worin

$R_1$ und $R_2$ unabhängig voneinander für Wasserstoff, Halogen, Methoxy oder Methylthio stehen ;

Z für eine der Gruppen

a) 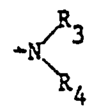 oder

b) 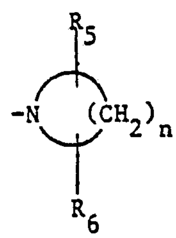 oder

c) 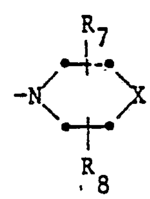

steht, wobei

$R_3$ und $R_4$ unabhängig voneinander für Wasserstoff, unsubstituiertes oder durch Cyano, $C_1$-$C_6$-Alkoxy oder $C_1$-$C_6$-Alkoxycarbonyl substituiertes $C_1$-$C_6$-Alkyl, $C_3$-$C_6$-Alkenyl, $C_3$-$C_6$-Alkinyl, $C_3$-$C_7$-Cycloalkyl, unsubstituiertes oder durch Halogen, $C_1$-$C_6$-Alkyl, $C_1$-$C_6$-Haloalkyl und/oder $C_1$-$C_6$-Alkoxy substituiertes Benzyl oder unsubstituiertes oder durch Halogen, $C_1$-$C_6$-Alkyl, $C_1$-$C_6$-Haloalkyl und/oder $C_1$-$C_6$-Alkoxy substituiertes Phenyl stehen, mit der Massgabe, dass nur $R_3$ oder $R_4$ Wasserstoff sein kann ;

$R_5$ und $R_6$ unabhängig voneinander für Wasserstoff, $C_1$-$C_6$-Alkyl, $C_1$-$C_6$-Alkoxycarbonyl stehen oder beide zusammen einen ankondensierten Phenylring bilden ;

n für eine der Zahlen 4 oder 5 steht ;

X Sauerstoff, Schwefel, $>C{=}O$ oder $>N{-}R_9$ bedeutet ;

$R_7$ und $R_8$ unabhängig voneinander für Wasserstoff, $C_1$-$C_6$-Alkyl oder $C_1$-$C_6$-Alkoxycarbonyl stehen ; und

$R_9$ Wasserstoff, $C_1$-$C_6$-Alkyl, Formyl, $C_1$-$C_6$-Alkanoyl oder $C_1$-$C_6$-Alkoxycarbonyl bedeutet.

Unter dem Begriff Alkyl selbst oder als Bestandteil eines anderen Substituenten sind je nach Zahl der angegebenen Kohlenstoffatome beispielsweise folgende Gruppen zu verstehen : Methyl, Ethyl, Propyl, Butyl, Pentyl, Hexyl usw., sowie ihre Isomeren, wie z. B. Isopropyl, Isobutyl, tert.-Butyl, Isopentyl usw.. Haloalkyl steht für einen einfach bis perhalogenierten Alkylsubstituenten, wie z. B. $CH_2Cl$, $CHCl_2$, $CCl_3$, $CH_2Br$, $CHBr_2$, $CBr_3$, $CH_2F$, $CHF_2$, $CF_3$, $CCl_2F$, $CCl_2$-$CHCl_2$, $CH_2CH_2F$, $CJ_3$ usw., vorzugsweise $CF_3$.

Unter Halogen soll hier und im folgenden Fluor, Chlor, Brom oder Jod, vorzugsweise Fluor, Chlor

oder Brom, verstanden werden. Alkenyl steht z. B. für Vinyl, Propenyl-(1), Allyl, Butenyl-(1), Butenyl-(2), Butenyl-(3), usw. sowie für Alkylketten, die durch mehrere C=C-Doppelbindungen unterbrochen sind. Alkinyl bedeutet z. B. Propinyl-(2), Propargyl, Butinyl-(1), Butinyl-(2) usw., vorzugsweise Propargyl. Cycloalkyl steht je nach Zahl der angegebenen Kohlenstoffatome z. B. für Cyclopropyl, Cyclobutyl, Cyclopentyl, Cyclohexyl, Cycloheptyl usw. Alkanoyl steht stellvertretend für eine an eine Carbonylgruppe gebundene, geradkettige oder verzweigte Alkylgruppe. Bilden $R_5$ und $R_6$ zusammen einen ankondensierten Phenylring, so steht Z vorzugsweise für folgende Gruppierung :

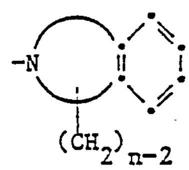

wobei n die unter Formel I angegebenen Bedeutungen hat.

Die Verbindungen der Formel I sind unter Normalbedingungen stabile Oele, Harze oder überwiegend kristalline Feststoffe, die sich durch äusserst wertvolle mikrobizide Eigenschaften auszeichnen. Sie lassen sich vor allem unter Freilandbedingungen auf dem Agrarsektor oder verwandten Gebieten kurativ und insbesondere präventiv zur Bekämpfung pflanzenschädigender Mikroorganismen einsetzen. Die erfindungsgemässen Wirkstoffe der Formel I zeichnen sich in weiten Anwendungskonzentrationen durch eine hohe fungizide Aktivität und problemlose Anwendung aus.

Aufgrund ihrer ausgeprägten mikrobiziden Aktivität sind folgende Substanzgruppen in steigender Reihenfolge zunehmend bevorzugt :

a) Verbindungen der Formel I, worin $R_1$ und $R_2$ unabhängig voneinander für Wasserstoff, Chlor, Methoxy oder Methylthio stehen ; Z für eine der Gruppen

a) 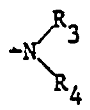 oder

b) 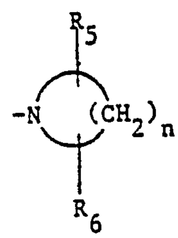 oder

c) 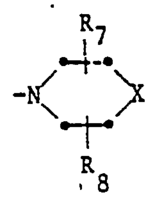

steht, wobei $R_3$ und $R_4$ unabhängig voneinander für Wasserstoff, $C_1$-$C_6$-Alkyl, durch Cyano, $C_1$-$C_3$-Alkoxy oder $C_1$-$C_3$-Alkoxycarbonyl substituiertes $C_1$-$C_3$-Alkyl, $C_3$-$C_4$-Alkenyl, $C_3$-$C_4$-Alkinyl, $C_5$-$C_6$-Cycloalkyl, unsubstituiertes oder durch Halogen, Methyl, Trifluormethyl und/oder Methoxy substituiertes Benzyl oder unsubstituiertes oder durch Halogen, Methyl, Trifluormethyl und/oder Methoxy substituiertes Phenyl stehen, mit der Massgabe, dass nur $R_3$ oder $R_4$ Wasserstoff sein kann ; $R_5$ und $R_6$ unabhängig voneinander für Wasserstoff, $C_1$-$C_3$-Alkyl oder $C_1$-$C_3$-Alkoxycarbonyl stehen oder beide zusammen einen ankondensierten Phenylring bilden ; n für eine der Zahlen 4 oder 5 steht ; X Sauerstoff, Schwefel, $>C=O$ oder $>N-R_9$ bedeutet ; $R_7$ und $R_8$ unabhängig voneinander für Wasserstoff, $C_1$-$C_3$-Alkyl oder $C_1$-$C_3$-Alkoxycarbonyl stehen ; und $R_9$ Wasserstoff, $C_1$-$C_3$-Alkyl, Formyl, $C_1$-$C_3$-Alkanoyl oder $C_1$-$C_3$-Alkoxycarbonyl bedeutet.

b) Verbindungen der Formel I, worin $R_1$ in der 2-Position und $R_2$ in der 3-Position fixiert sind und unabhängig voneinander für Wasserstoff, Chlor, Methoxy oder Methylthio stehen ;

Z für eine der Gruppen

a) 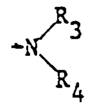 oder

b) 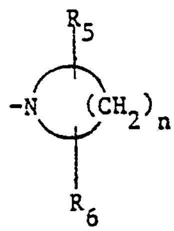 oder

c) 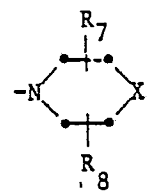

steht, wobei $R_3$ und $R_4$ unabhängig voneinander für $C_1$-$C_6$-Alkyl, durch Cyano, $C_1$-$C_3$-Alkoxy oder $C_1$-$C_3$-Alkoxycarbonyl substituiertes $C_1$-$C_2$-Alkyl, Allyl, Propargyl, Cyclopentyl, Cyclohexyl, Benzyl, Halobenzyl, Phenyl oder Halophenyl stehen ; $R_5$ und $R_6$ unabhängig voneinander für Wasserstoff, Methyl oder $C_1$-$C_3$-Alkoxycarbonyl stehen oder beide zusammen einen ankondensierten Phenylring bilden ; n für eine der Zahlen 4 oder 5 steht ; X Sauerstoff, Schwefel, $>C{=}O$ oder $>N{-}R_9$ bedeutet ; $R_7$ und $R_8$ unabhängig voneinander für Wasserstoff, $C_1$-$C_2$-Alkyl, Formyl, $C_1$-$C_3$-Alkanoyl oder $C_1$-$C_3$-Alkoxycarbonyl bedeutet.

c) Verbindungen der Formel I, worin $R_1$ für Wasserstoff oder 2-Chlor und $R_2$ für Wasserstoff oder 3-Chlor stehen ;

Z für eine der Gruppen

a) 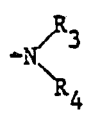 oder

b) 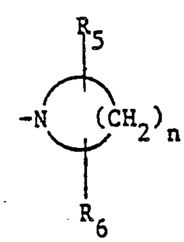 oder .

c) 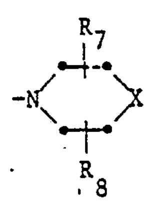

steht, wobei $R_3$ und $R_4$ unabhängig voneinander für $C_1$-$C_6$-Alkyl, durch Cyano, $C_1$-$C_3$-Alkoxy oder $C_1$-$C_3$-Alkoxycarbonyl substituiertes $C_1$-$C_2$-Alkyl, Allyl, Propargyl, Cyclopentyl, Cyclohexyl, Benzyl, Halobenzyl, Phenyl oder Halophenyl stehen ; $R_5$ und $R_6$ unabhängig voneinander für Wasserstoff, Methyl oder $C_1$-$C_3$-Alkoxycarbonyl stehen oder beide zusammen einen ankondensierten Phenylring bilden ; n für eine der Zahlen 4 oder 5 steht ; X Sauerstoff, Schwefel, $>C{=}O$ oder $>N{-}R_9$ bedeutet ; $R_7$ und $R_8$ unabhängig voneinander für Wasserstoff, $C_1$-$C_2$-Alkyl, Formyl, $C_1$-$C_3$-Alkanoyl oder $C_1$-$C_3$-Alkoxycarbonyl bedeutet.

Besonders bevorzugte Einzelsubstanzen sind beispielsweise :
N-(N',N'-Dimethylaminomethyl)-3-(2,3-dichlorphenyl)-4-cyanopyrrol (Nr. 1.1),
N-(N'-Benzyl-N'-methylaminomethyl)-3-(2,3-dichlorphenyl)-4-cyanopyrrol (Nr. 1.9),
N-[N'-Methyl-N'-(2-cyanoethyl)-aminomethyl]-3-(2,3-dichlorphenyl)-4-cyanopyrrol (Nr. 1.16),
N-[N'-Isopropyl-N'-(2-cyanoethyl)-aminomethyl]-3-(2,3-dichlorphenyl)-4-cyanopyrrol (Nr. 1.23),
N-(Piperidin-1-ylmethyl)-3-(2,3-dichlorphenyl)-4-cyanopyrrol (Nr. 1.45),
N-(3-Ethoxycarbonylpiperidin-1-ylmethyl)-3-(2-chlorphenyl)-4-cyanopyrrol (Nr. 1.48),
N-(Morpholin-1-ylmethyl)-3-(2,3-dichlorphenyl)-4-cyanopyrrol (Nr. 1.55),
N-(2,6-Dimethylmorpholin-1-ylmethyl)-3-(2-chlorphenyl-4-cyanopyrrol (Nr. 1.61),
N-[N'-Cyclopentyl-N'-(2-cyanoethyl)-aminomethyl]-3-(2,3-dichlorphenyl)-4-cyanopyrrol (Nr. 1.29),
N-(2,6-Dimethylmorpholin-1-ylmethyl)-3-(2,3-dichlorphenyl)-4-cyanopyrrol (Nr. 1.63/1.75),
N-(4-Acetylcarbonylpiperazin-1-ylmethyl)-3-(2,3-dichlorphenyl)-4-cyanopyrrol (Nr. 1.68),
N-[N'-(2-Methoxyethyl)-N'-(2-cyanoethyl)-aminomethyl]-3-(2,3-dichlorphenyl)-4-cyanopyrrol (Nr. 1.34),
N-[N'-Methyl-N'-(2-cyanoethyl)-aminomethyl]-3-(2-chlorphenyl)-4-cyanopyrrol (Nr. 1.11),
N-[N'-Methyl-N'-(2-methoxyethyl)-aminomethyl]-3-(2,3-dichlorphenyl)-4-cyanopyrrol (Nr. 1.14),
N-[N'-(n-Propyl)-N'-(2-cyanoethyl)-aminomethyl]-3-(2,3-dichlorphenyl)-4-cyanopyrrol (Nr. 120),
N-[N'-(n-Butyl)-N'-(2-cyanoethyl)-aminomethyl]-3-(2,3-dichlorphenyl)-4-cyanopyrrol (Nr. 1.21),
N-[N'-Methyl-N'-prop-2-enyl-aminomethyl]-3-(2,3-dichlorphenyl)-4-cyanopyrrol (Nr. 1.70),
N-[N'-Propargyl-N'-cyclohexyl-aminomethyl]-3-(2,3-dichlorphenyl)-4-cyanopyrrol (Nr. 1.72).

Die Verbindungen der Formel I werden erfindungsgemäss folgendermassen hergestellt :

A) indem man eine Verbindung der Formel II

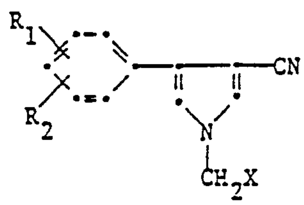

(II)

worin $R_1$ und $R_2$ die unter Formel I angegebenen Bedeutungen haben und X für OH oder Halogen, vorzugsweise Chlor, steht, in Gegenwart einer Base und gegebenenfalls im Beisein eines Reaktionskatalysators bei bei Temperaturen von 0° bis 100 °C, bevorzugt 20° bis 60 °C mit einer Verbindung der Formel III

$$H—Z \quad (III)$$

umsetzt, worin Z die unter Formel I angegebenen Bedeutungen hat ; oder

B) indem man eine Verbindung der Formel IV

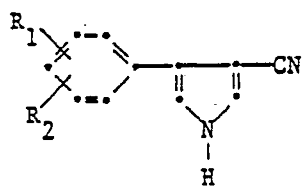

(IV)

worin $R_1$ und $R_2$ die unter Formel I angegebenen Bedeutungen haben, entweder in einem protischen Lösungsmittel bei Temperaturen von 0° bis 120 °C, vorzugsweise bei 20 °C bis Rückflusstemperatur, in Gegenwart eines basischen oder sauren Reaktionskatalysators mit Formaldehyd und einer Verbindung der Formel III umsetzt oder in einem aprotischen Lösungsmittel im Beisein eines basischen Reaktionskatalysators und bei Temperaturen von 0° bis 120 °C, vorzugsweise 20° bis 80 °C, mit 1,3,5-Trioxan oder Paraformaldehyd und einer Verbindung der Formel III umsetzt.

Die Variante A (Reaktion von II mit III) wird in Ab- oder vorzugsweise Anwesenheit reaktionsinerter Lösungsmittel oder Lösungsmittelgemische durchgeführt. Beispiele solcher Lösungsmittel sind : Aromatische und aliphatischen Kohlenwasserstoffe wie Benzol, Toluol, Xylole, Petrolether, Ligroin oder Cyclohexan ; halogenierte Kohlenwasserstoffe wie Chlorbenzol, Methylenchlorid, Ethylenchlorid, Chloroform, Tetrachlorkohlenstoff oder Tetrachlorethylen ; Ether und etherartige Verbindungen wie Diethylether, Diisopropylether, T-Butylmethylether, Dimethoxyethan, Dioxan, Tetrahydrofuran oder Anisol ; Ester wie Ethylacetat, Propylacetat oder Butylacetat ; Nitrile wie Acetonitril ; oder Verbindungen wie Dimethylsulfoxid, Dimethylformamid oder Gemische solcher Lösungsmittel untereinander.

Durch Zusatz eines Reaktionskatalysators, wie z. B. KJ, kann die Reaktionsgeschwindigkeit in einigen Fällen erhöht werden. Basen werden bei dieser Reaktionsvariante A üblicherweise in äquimolaren

Mengen zugesetzt. Geeignete Basen sind z. B. organische Basen wie Trialkylamine (Trimethylamin, Triethylamin usw.). Pyridin und Pyridinbasen oder anorganische Basen wie Hydrogencarbonate oder Carbonate von Alkali- und Erdalkalimetallen. Im allgemeinen kann auch überschüssiges Amin der Formel III als Base eingesetzt werden.

Die Reaktionsvariante B (Reaktion von III mit IV) wird vorzugsweise in einem geeigneten reaktionsinerten Lösungsmittel durchgeführt. Geeignete protische Lösungsmittel sind z. B.: Wasser, Alkohole (insbesondere Alkanole, wie Methanol, Ethanol, Isopropanol, n-Propanol usw.) oder Carbonsäuren (insbesondere Alkancarbonsäuren, wie Ameisensäure, Essigsäure, Propionsäure usw.). Arbeitet man in einem protischen Lösungsmittel, so können als Reaktionskatalysatoren z. B. folgende Substanzen eingesetzt werden: Organische Basen wie z. B. 1,8-Diazabicyclo [5.4.0]-undec-7-en, tertiäre Amine wie Trialkylamine, (Trimethylamin, Triethylamin, Dimethylethylamin, usw.), Triethylendiamin, Piperidin, Pyridin, 4-Dimethylaminopyridin, 4-Pyrrolidylpyridin usw. oder anorganische Basen wie die Oxide, Hydroxide, Hybride, Carbonate, Hydrogencarbonate und Alkoholate von Alkali- oder Erdalkalimetallen (z. B. $Na_2CO_3$, $BaCO_3$, $MgCO_3$, $K_2CO_3$, $Na_2HCO_3$, $K_2HCO_3$, $CaHCO_3$, $NaOCH_3$, $NaOC_2H_5$, KO(iso-$C_3H_7$), KO(t.-Butyl), NaH, CaO usw.). Organische Säuren wie z. B. Carbonsäuren (Essigsäure, Ameisensäure, Propionsäure, usw.), aliphatische und aromatische Sulfonsäuren wie Methansulfonsäure, Ethansulfonsäure, Toluolsulfonsäure, Benzolsulfonsäure, usw. Anorganische Säuren wie Mineralsäuren, z. B. Phosphonsäure, Schwefelsäure, Salpetersäure oder Halogenwasserstoffsäuren (Chlorwasserstoffsäure, Bromwasserstoffsäure, Jodwasserstoffsäure oder Fluorwasserstoffsäure). Säuren oder Basen werden bei dieser Reaktionsvariante vorteilhafterweise in katalytischen Mengen zugesetzt. Im allgemeinen genügt bereits ein Ueberschuss des Amins der Formel III. Der Formaldehyd wird bei dieser Variante vorzugsweise in Form seiner wässrigen Lösung (Formalin) oder als Trimeres (1,3,5-Trioxan) oder als Polymeres (Paraformaldehyd) eingesetzt.

Arbeitet man bei der Reaktionsvariante B in einem aprotischen Lösungsmittel (es eignen sich z. B. aliphatische oder aromatische Kohlenwasserstoffe wie Benzol, Toluol, Xylole, Petrolether, Ligroin, Benzine oder Cyclohexan, Ether und etherartige Verbindungen wie Diethylether, Diisopropylether, Tert.-Butylmethylether, Dimethoxyethan, Tetrahydrofuran, Dioxan oder Anisol, Ester wie Ethylacetat, Propylacetat oder Butylacetat, oder Verbindungen wie Dimethylformamid, Dimethylsulfoxid und Gemisch solcher Lösungsmittel untereinander. Als katalysatoren werden z. B. die unter Variante B weiter oben aufgeführten Basen eingesetzt. Bei dieser Herstellungsvariante wird der Formaldehyd vorzugsweise als 1,3,5-Trioxan oder Paraformaldehyd eingesetzt. In beiden Varianten A oder B wird im allgemeinen mit 1,0 bis 1,5 Aequivalenten Formaldehyd umgesetzt. Amine der Formel III sind bekannt oder werden nach an sich bekannten Methoden hergestellt.

Die Ausgangsprodukte der Formel II sind aus den zugrundeliegenden 3-Phenyl-4-cyanopyrrolen der Formel IV erhältlich, z. B. dadurch, dass man eine Verbindung der Formel IV durch Reaktion mit Formaldehyd in ein das entsprechende N-Hydroxymethyl-Derivat der Formel V

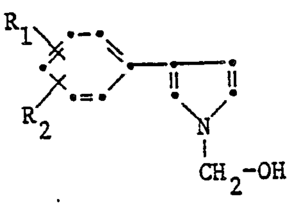

(V)

worin $R_1$ und $R_2$ die unter Formel I angegebenen Bedeutungen haben, überführt und sofern erforderlich, die freie OH-Gruppe in V auf übliche Weise durch ein Halogen austauscht, wobei der Austausch z. B. mit Thionylchlorid oder Phosphoroxychlorid erfolgen kann.

Die Herstellung von Verbindungen der Formel V erfolgt im allgemeinen bei Temperaturen zwischen 0° und 200 °C, vorzugsweise 0° und 160 °C, in einem üblichen reaktionsinerten organischen Lösungsmittel oder Lösungsmittelgemisch.

Ein Teil der Verbindungen der Pyrole der Formel IV ist aus der Literatur bekannt. So werden z. B. die Herstellungsmethode und die chemischen Eigenschaften von 4-Cyano-3-phenyl-pyrrol in Tetrahedron-Letters No. 52, S. 5337-5340 (1972) beschrieben. Ueber eine biologische Wirksamkeit der Verbindung wird nicht berichtet. Andere Vertreter der Formel IV lassen sich analog zu den bekannten Verbindungen herstellen.

Unterschiedlich substituierte 3-Phenyl-4-cyanopyrrol-derivate sind aus der Literatur bekannt, so werden beispielsweise Pyrrole der Formel VI

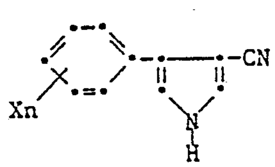

(VI)

worin X ein Halogenatom, eine niedere Alkyl- oder niedere Halogenalkylgruppe bedeutet und n für 0, 1 oder 2 steht, in der DE-OS 2,927,480 als Zwischenprodukte mit geringer fungizider Aktivität beschrieben.

Ferner sind Pyrrol-derivate der Formel VII

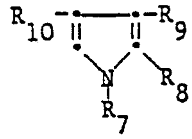

(VII)

mit

$R_7$ = Acyl, Alkoxycarbonylalkyl...

$R_8$, $R_9$ = H, Aryl...

$R_{10}$ = Hydroxy, Mercapto

aus der GB-PS 2,078,761 als Hitze- und Lichtstabilisatoren für PVC-Kunststoffe bekannt.

Weitere Pyrrol-derivate der Formel VIII

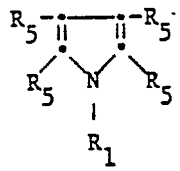

(VIII)

mit

$R_1$ = Alkyl, gegebenenfalls durch Acyloxy substituiert...

$R_5$ = H, Alkyl, CN...

werden als Polymerisationskatalysatoren für Vinylchlorid in der DE-OS 2,028,363 genannt.

Die bekannten Pyrrol-derivate sind entweder gegenüber Pflanzenpathogenen unwirksam oder zeigen zwar im Gewächshaus eine deutliche fungizide Wirkung, die jedoch unter Freilandbedingungen auf Grund ihrer Instabilität gegenüber Umwelteinflüssen nicht reproduzierbar ist. Sie sind daher für den praktischen Einsatz in der Landwirtschaft, im Gartenbau oder in verwandten Einsatzgebieten nicht geeignet. Die erfindungsgemässen, neuen Pyrrolderivate der Formel I stellen dagegen eine wertvolle Bereicherung des Standes der Technik dar, denn es wurde überraschend festgestellt, dass die neuen Pyrrole der Formel I ein für Freiland-Bedürfnisse sehr günstiges Mikrobizid-Spektrum gegen phytopathogene Pilze und Bakterien aufweisen. Sie können aber nicht nur im Ackerbau oder ähnlichen Einsatzgebieten zur Bekämpfung schädlicher Mikroorganismen an Kulturpflanzen, sondern zusätzlich im Vorratsschutz zur Konservierung verderblicher Waren eingesetzt werden. Verbindungen der Formel I besitzen sehr vorteilhafte kurative, systemische und insbesondere präventive Eigenschaften und lassen sich zum Schutz von zahlreichen, insbesondere Freilandkulturen einsetzen. Mit den Wirkstoffen der Formel I können an Pflanzen oder an Pflanzenteilen (Früchte, Blüten, Laubwerk, Stengel, Knollen, Wurzeln) von unterschliedlichen Nutzkulturen die auftretenden Mikroorganismen eingedämmt oder vernichtet werden, wobei auch später zuwachsende Pflanzenteile von derartigen Mikroorganismen verschont bleiben.

Die Wirkstoffe sind beispielsweise gegen die den folgenden Klassen angehörenden phytopathogenen Pilze wirksam: Ascomycetes z. B. Erysiphe, Sclerotinia, Fusarium, Monilinia, Helminthosporium; Basidiomycetes wie z. B. Puccinia, Tilletia, Rhizoctonia; sowie die der Klasse der Phycomycetes angehörenden Oomycetes wie Phytophthora. Als Pflanzenschutzmittel können die Verbindungen der Formel I mit besonders gutem Erfolg gegen wichtige Schadpilze aus der Familie der Fungi imperfecti eingesetzt werden, so z. B. gegen Cercospora, Piricularia und vor allem gegen Botrytis. Botrytis spp. (B. cinerea, B. allii) stellen mit der Grauschimmelfäule an Reben, Erdbeeren, Aepfeln, Zwiebeln und anderen Obst- und Gemüsesorten einen bedeutenden wirtschaftlichen Schadfaktor dar. Darüberhinaus lassen sich einige Verbindungen der Formel I erfolgreich zum Schutz verderblicher Waren pflanzlicher oder tierischer Herkunft einsetzen. Sie bekämpfen Schimmelpilze wie Penicillium, Aspergillus, Rhizopus, Fusarium, Helminthosporium, Nigrospora und Alternaria sowie Bakterien wie Buttersäurebakterien und Hefen wie Candida.

Als Pflanzenschutzmittel besitzen die Verbindungen der Formel I ein für die praktische Anwendung in der Landwirtschaft sehr günstiges Wirkungsspektrum zum Schutze von Kulturpflanzen, ohne diese durch unerwünschte Nebenwirkungen nachteilig zu beeinflussen.

Sie können ferner als Beizmittel zur Behandlung von Saatgut (Früchte, Knollen, Körner) und Pflanzenstecklingen zum Schutz vor Pilzinfektionen sowie gegen im Erdboden auftretende phytopathogene Pilze eingesetzt werden.

Die Erfindung betrifft somit auch mikrobizide Mittel sowie die Verwendung der Verbindungen der Formel I zur Bekämpfung phytopathogener Mikroorganismen, insbesondere pflanzenschädigender Pilze bzw. die präventive Verhütung eines Befalls an Pflanzen und an Vorräten pflanzlicher oder tierischer Herkunft.

Darüberhinaus schliesst die vorliegende Erfindung auch die Herstellung (agro)chemischer Mittel ein, die gekennzeichnet ist durch das innige Vermischen der Aktivsubstanz mit einem oder mehreren hierin beschriebenen Substanzen bzw. Substanzgruppen. Eingeschlossen ist auch ein Verfahren zur Behandlung von Pflanzen oder Vorratsgütern, das sich durch Applikation der Verbindungen der Formel I bzw. der neuen Mittel auf die Pflanzen, Pflanzenteile, deren Standort oder das Substrat kennzeichnet.

Als Zielkulturen für den Pflanzenschutz gelten im Rahmen dieser Erfindung beispielsweise folgende Pflanzenarten: Getreide: (Weizen, Gerste, Roggen, Hafer, Reis, Sorghum und Verwandte); Rüben: (Zucker- und Futterrüben); Kern-, Stein- und Beerenobst: (Aepfel, Birnen, Pflaumen, Pfirsiche, Mandeln, Kirschen, Erd-, Him- und Brombeeren); Hülsenfrüchte: (Bohnen, Linsen, Erbsen, Soja); Oelkulturen: (Raps, Senf, Mohn, Oliven, Sonnenblumen, Kokos, Rizinus, Kakao, Erdnüssen); Gurkengewächse: (Kürbis, Gurken, Melonen); Fasergewächse: (Baumwolle, Flachs, Hanf, Jute); Citrusfrüchte: (Orangen, Zitronen, Pampelmusen, Mandarinen); Gemüsesorten: (Spinat, Kopfsalat, Spargel, Kohlarten, Möhren, Zwiebeln, Tomaten, Kartoffeln, Paprika); Lorbeergewächse: (Avocado, Cinnamonum, Kampfer) oder Pflanzen wie Mais, Tabak, Nüsse, Kaffee, Zuckerrohr, Tee, Weinreben, Hopfen, Bananen- und Naturkautschukgewächse sowie Zierpflanzen (Compositen).

Als Vorratsschutzmittel werden die Verbindungen der Formel I in unveränderter Form oder vorzugsweise zusammen mit den in der Formulierungstechnik üblichen Hilfsmitteln eingesetzt und werden z. B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z. B. polymeren Stoffen in bekannter Weise verarbeitet. Die Anwendungsverfahren wie Versprühen, Vernebeln, Verstäuben, Verstreuen, Bestreichen oder Giessen und die Form des Mittels werden den angestrebten Zielen und den gegebenen Verhältnissen angepasst. Günstige Aufwandmengen liegen im allgemeinen bei 0,01 bis höchstens 2 kg Aktivsubstanz pro 100 kg zu schützendes Substrat; sie hängen jedoch ganz wesentlich von der Beschaffenheit (Grösse der Oberfläche, Konsistenz, Feuchtigkeitsgehalt) des Substrats und dessen Umgebungseinflüssen ab.

Unter Lager- und Vorratsgütern sollen im Rahmen vorliegender Erfindung pflanzliche und/oder tierische Naturstoffe und deren Weiterverarbeitungsprodukte verstanden werden, beispielsweise die nachfolgend aufgezählten und aus dem natürlichen Lebenzyklus herausgenommenen Pflanzen, deren Pflanzenteile (Stengel, Blätter, Knollen, Samen, Früchte, Körner), die im frisch geernteten Zustand oder in weiterverarbeitbarer Form vorliegen (vorgetrocknet, befeuchtet, zerkleinert, gemahlen, geröstet). Als Beispiele, die keinen das Anwendungsgebiet limitierenden Charakter im Rahmen dieser Erfindung besitzen, seien folgende Ertragsgüter genannt: Getreide (wie Weizen, Gerste, Roggen, Hafer, Reis, Sorghum und Verwandte); Rüben (wie Möhren, Zucker- und Futterrüben); Kern-, Stein- und Beerenobst (wie Aepfel, Birnen, Pflaumen, Pfirsiche, Mandeln, Kirschen, Erd-, Him- und Brombeeren); Hülsenfrüchte (wie Bohnen, Linsen, Erbsen, Soja); Oelkulturen (wie Raps, Senf, Mohn, Oliven, Sonnenblumen, Kokos, Rizinus, Kakao, Erdnüsse); Gurkengewächse (wie Kürbis, Gurken, Melonen); Fasergewächse (wie Baumwolle, Flachs, Hanf, Jute, Nessel); Citrusfrüchte; Gemüsesorten (wie Spinat, Salat, Spargel, Kohlarten), Zwiebeln, Tomaten, Kartoffeln, Paprika): Lorbeergewächse (wie Avocadom Cinnamonum, Kampfer) oder Mais, Tabak, Nüsse, Kaffee, Zuckerrohr, Tee, Weintrauben, Kastanien, Hopfen, Bananen, Gras und Heu.

Als Naturprodukte tierischer Herkunft seien insbesondere getrocknete Fleisch- und Fischverarbeitungsprodukte wie Trockenfleisch, Trockenfisch, Fleischkonzentrate, Knochenmehl, Fischmehl und Tiertrockenfutter genannt.

Das behandelte Vorratsgut wird durch Behandlung mit Verbindungen der Formel I nachhaltig vor dem Befall durch Schimmelpilze und andere unerwünschte Mikroorganismen geschützt. Dadurch wird die Bildung toxischer und zum Teil karzinogener Schimmelpilze (Aflatoxine und Ochratoxine) unterbunden, das Gut vor dem Verderben bewahrt und dessen Qualität für längere Zeit aufrechterhalten. Das erfindungsgemässe Verfahren kann auf alle trockenen und feuchten Vorrats- und Lagergüter angewendet werden, die gegen Mikroorganismen, wie Hefen, Bakterien und insbesondere Schimmelpilze anfällig sind.

Ein bevorzugtes Verfahren zum Aufbringen des Wirkstoffes besteht in einem Besprühen oder Benetzen des Substrats mit einer flüssigen Aufbereitung oder im Vermischen des Substrats mit einer festen Aufbereitung der Aktivsubstanz. Das beschriebene Konservierungs-Verfahren ist ein Teil der vorliegenden Erfindung.

Wirkstoffe der Formel I werden üblicherweise in Form von Zusammensetzungen verwendet und können gleichzeitig oder nacheinander mit weiteren Wirkstoffen auf die zu behandelnde Fläche, Pflanze oder Substrat gegeben werden. Diese weiteren Wirkstoffe können sowohl Düngemittel, Spurenelement-Vermittler oder andere das Pflanzenwachstum beeinflussende Präparate sein. Es können aber auch selektive Herbizide, Insektizide, Fungizide, Bakterizide, Nematizide, Molluskizide oder Gemische mehrerer dieser Präparate sein, zusammen mit gegebenenfalls weiteren in der Formulierungstechnik üblichen Trägerstoffen, Tensiden oder anderen applikationsfördernden Zusätzen.

Geeignete Träger und Zusätze können fest oder flüssig sein und entsprechen den in der Formulierungstechnik zweckdienlichen Stoffen, wie z. B. natürlichen oder regenerierten mineralischen Stoffen, Lösungs-, Dispergier, Netz-, Haft-, Verdickungs-, Binde- oder Düngemitteln.

Ein bevorzugtes Verfahren zum Aufbringen eines Wirkstoffes der Formel I bzw. eines (agro)chemischen Mittels, das mindestens einen dieser Wirkstoffe enthält, ist das Aufbringen auf das Blattwerk (Blattapplikation). Anzahl der Applikationen und Aufwandmenge richten sich dabei nach dem Befallsdruck für den entsprechenden Erreger (Pilzsorte). Die Wirkstoffe der Formel I können aber auch über den Erdboden durch das Wurzelwerk in die Pflanze gelangen (systemische Wirkung), indem man den Standort der Pflanze mit einer flüssigen Zubereitung tränkt oder die Substanzen in fester Form in den Boden einbringt, z. B. in Form von Granulat (Bodenapplikation). Die Verbindungen der Formel I können aber auch auf Samenkörner aufgebracht werden (Coating), indem man die Körner entweder mit einer flüssigen Zubereitung des Wirkstoffs tränkt oder sie mit einer festen Zubereitung beschichtet. Darüberhinaus sind in besonderen Fällen weitere Applikationsarten möglich, so z. B. die gezielte Behandlung der Pflanzenstengel oder der Knospen.

Die Verbindungen der Formel I werden dabei in unveränderter Form oder vorzugsweise zusammen mit den in der Formulierungstechnik üblichen Hilfsmittel eingesetzt und werden daher z. B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z. B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt. Günstige Aufwandmengen liegen im allgemeinen bei 50 g bis 5 kg Aktivsubstanz (AS) je ha; bevorzugt 100 g bis 2 kg AS/ha, insbesondere bei 200 g bis 600 g AS/ha.

Die Formulierungen d. h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z. B. durch inniges Vermischen und/oder Vermahlen der Wirkstoffe mit Streckmitteln, wie z. B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z. B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol Ethylenglykol, Ethylenglykolmonomethyl- oder -ethylether, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl, Sonnenblumenöl oder Sojaöl; oder Wasser.

Als feste Trägerstoffe, z. B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z. B. Bimsstein, Ziegelbruch, Sepiolit oder Bentonit, als nicht adsorptive Trägermaterialien z. B. Calcit oder Sand in Frage, Darüberhinaus kann eine Vielzahl von vorgranulierten Materialien anorganischer oder organischer Natur wie insbesondere Dolomit oder zerkleinerte Pflanzenrückstände, wie z. B. Korkmehl oder Sägemehl, verwendet werden.

Besonders vorteilhafte, applikationsfördernde Zuschlagstoffe, die zu einer starken Reduktion der Aufwandmenge führen können, sind ferner natürliche (tierische oder pflanzliche) oder synthetische Phospholipide aus der Reihe der Kephaline und Lecithine, wie z. B. Phosphatidylethanolamin, Phosphatidylserin, Phosphatidylcholin, Sphingomyelin, Phosphatidylinosit, Phosphatidylglycerin, Lysolecithin, Plasmalogene oder Cardiolipin, die man beispielsweise aus tierischen oder pflanzlichen Zellen, insbesondere Hirn, Herz, Lunge, Leber, Eidotter oder Sojabohnen gewinnen kann. Verwendbare Handelsmischungen sind z. B. Phosphatidylcholin-Mischungen. Synthetische Phospholipide sind z. B. Dioctanoylphosphatidylcholin und Dipalmititoylphosphatidylcholin.

Als oberflächenaktive Verbindungen kommen je nach Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen, als auch wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$-$C_{22}$), wie z. B. die Na- oder K-Salze der Oel- oder Stearinsäure, oder von natürlichen Fettsäuregemischen, die z. B. aus Kokosnuss- oder Talgöl gewonnen werden können. Ferner sind auch die Fettsäure-methyllaurinsalze zu erwähnen.

Häufiger werden jedoch sog. synthetische Tenside verwendet, insbesondere Fettsulfonate, Fettsulfate, sulfonierte Benzimidazolderivate oder Alkylsulfonate.

Die Fettsulfonate oder -sulfate liegen in der Regel als Alkali-, Erdalkali- oder gegebenenfalls substituierte Ammoniumsalze vor und weisen einen Alkalirest mit 8 bis 22 C-Atomen auf, wobei Alkyl auch den Alkylteil von Acylresten einschliesst, z. B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxid-Addukten. Die sulfonierten Benzimidazolderivate enthalten vorzugsweise 2-Sulfonsäuregruppen und einen Fettsäurerest mit 8-22 C-Atomen. Alkylarylsulfonate sind z. B. die Na-, Ca- oder

Triethanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z. B. Salze des Phosphorsäureesters eines p-Nonylphenol-4-14-ethylenoxidaddukte in Frage.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Ethylenglykolethergruppen und 10 bis 100 g Propylenglykolethergruppen enthaltenden Polyethylenoxidaddukte an Polypropylenglykol, Ethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykoleinheiten.

Als Beispiele nichtionischer Tenside seien Nonylphenolpolyethoxyethanole, Ricinusölpolyglykolether, Polypropylen-Polyethylenoxidaddukte, Tributylphenoxypolyethylenethanol, Polyethylenglykol und Octylphenoxypolyethoxyethanol erwähnt.

Ferner kommen auch Fettsäureester von Polyoxyethylensorbitan wie das Polyoxyethylensorbitantrioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z. B. das Stearyltrimethylammoniumchlorid oder das Benzyldi(2-chlorethyl)ethylammoniumbromid. Auf dem Vorratsschutzgebiet werden die für die menschliche und tierische Ernährung unbedenklichen Zuschlagstoffe bevorzugt.

Die in der Formulierungstechnik gebräuchlichen Tenside sind u. a. in folgenden Publikationen beschrieben : « Mc Cutcheon's Detergents and Emulsifiers Annual » BC Publishing Corp., Ridgewood New Jersey, 1981. Helmut Stache « Tensid-Taschenbuch » Carl Hanser-Verlag, München/Wien 1981.

Die agrochemische Zubereitungen enthalten in der Regel 0,1 bis 99 %, insbesondere 0,1 bis 95 % Wirkstoff der Formel I, 99,9 bis 1 %, insbesondere 99,8 bis 5 % eines festen oder flüssigen Zusatzstoffes und 0 bis 25 %, insbesondere 0,1 bis 25 % eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Derartige (agro)chemische Mittel sind ein Bestandteil der vorliegenden Erfindung.

Die nachfolgenden Beispiele dienen zur näheren Erläuterung der Erfindung, ohne dieselbe einzuschränken. Temperaturen sind in Celsiusgraden angegeben. Prozente und Teile beziehen sich auf das Gewicht. Darüberhinaus werden folgende Symbole verwendet : h = Stunde ; d = Tag ; min = Minute ; RT = Raumtemperatur ; N = Normalität ; abs = absolut, wasserfrei ; DMSO = Dimethylsulfoxid ; DMF = Dimethylformamid. Druckangaben erfolgen in Millibar mb, oder Bar b. THF steht für Tetrahydrofuran.

## Herstellungsbeispiele

### α) Herstellung der Ausgangsprodukte

Beispiel A1 : Herstellung von

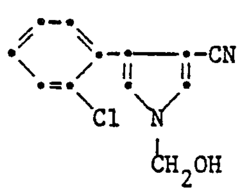

(Verb. Nr. 1)

N-Hydroxymethyl-3-(2-chlorphenyl)-4-cyanopyrrol

60,8 g 3-(2-chlorphenyl)-4-cyanopyrrol, 9,9 g Paraformaldehyd und 0,8 g Triethylamin werden gut vermischt und anschliessend unter Rühren bei 90 °C Badtemperatur erwärmt. Die entstandene Schmelze wird nach 1 h 15 min auf RT abgekühlt, wobei diese glasartig erstarrt. Die Umkristallisation aus Toluol liefert das obige Produkt in Form bräunlicher Kristalle. Smp. 94-97 °C.

Auf analoge Weise werden auch die nachfolgenden Verbindungen der Formel

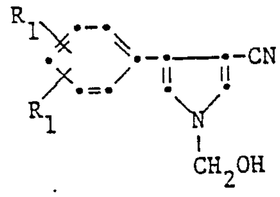

hergestellt:

| Verb. Nr. | $R_1$ | $R_2$ | Smp. [°C] |
|---|---|---|---|
| 1 | 2-Cl | H | 94-97 |
| 2 | H | 3-Cl | 72-74 |
| 3 | 2-Cl | 3-Cl | 137-140 |
| 4 | $2\text{-}OCH_3$ | H | zähe Masse |
| 5 | H | H | 81-84 |
| 6 | $2\text{-}SCH_3$ | H | Harz |

Beispiel A2: Herstellung von

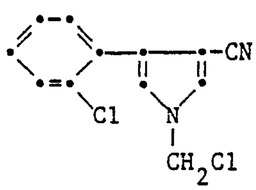

(Verb. Nr. 7)

N-Chlormethyl-3-(2-chlorphenyl)-4-cyanopyrrol

Zu 60 ml Thionylchlorid werden unter kräftigem Rühren 48 g N-Hydroxymethyl-3-(2-chlorphenyl)-4-cyanopyrrol in mehreren Portionen derart zugegeben, dass eine mässige Gasentwicklung aufrechterhalten bleibt. Nach Beendigung der Gasentwicklung wird das Gemisch noch 2 h bei RT und anschliessend 2,5 h bei 35-40 °C gerührt. Nach Abkühlen auf RT wird Toluol zugegeben und die Mischung eingeengt. Der Rückstand wird aus Diethylether/Petroleumbenzin umkristallisiert und liefert obiges Produkt in Form beiger Kristalle. Smp. 97-99 °C.

Beispiel A3: Herstellung von

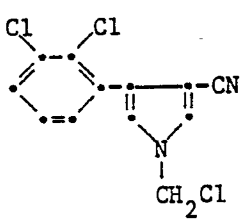

(Verb. Nr. 9)

N-Chlormethyl-3-(2,3-dichlorphenyl)-4-cyanopyrrol

Zu 23,7 g 3-(2,3-Dichlorphenyl)-4-cyanopyrrol gelöst in 300 ml Tetrahydrofuran werden 7,5 g Paraformaldehyd und anschliessend 1,5 ml 1,8-Diazabicyclo [5.3.0] undec-7-en gegeben. Es wird während 3 Std. bei Raumtemperatur gerührt. Anschliessend werden unter Rühren 21,7 ml Thionylchlorid bei 20-30° zum entsprechenden Hydroxymethyl-Derivat getropft. Das Reaktionsgemisch wird während 16

Std. bei Raumtemperatur gerührt, anschliessend auf Eiswasser gegossen und das resultierende Gemisch zweimal mit Essigsäureethylester extrahiert. Die vereinigten organischen Extrakte werden noch zweimal mit halbgesättigter Natriumchlorid-Lösung gewaschen, über Natriumsulfat getrocknet, filtriert und eingeengt. Kristallisation des Rückstandes aus Ether/Petroleumbenzin liefert das obige Produkt in Form beiger Kristalle. Smp. 132-133 °C.

Auf analoge Weise werden auch die nachfolgenden Verbindungen der Formel

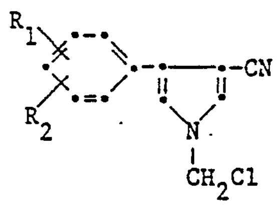

hergestellt:

| Verb. Nr. | $R_1$ | $R_2$ | Smp. [°C] |
|---|---|---|---|
| 7 | 2-Cl | H | 97-99 |
| 8 | H | 3-Cl | 77-79 |
| 9 | 2-Cl | 3-Cl | 132-133 |
| 10 | $2\text{-}OCH_3$ | H | |
| 11 | H | H | |
| 12 | $2\text{-}SCH_3$ | H | |

β Herstellung der Endprodukte

Beispiel H1 : Herstellung von

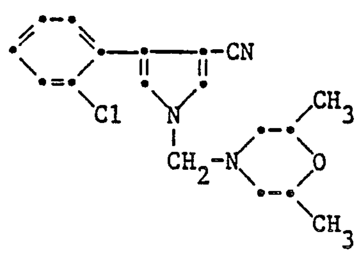

(Verb. Nr. 1.61)

N-(2,6-Dimethylmorpholin-4-ylmethyl)-3-(2-chlorphenyl)-4-cyanopyrrol

10 g N-Chlormethyl-3-(2-Chlorphenyl)-4-cyanopyrrol, 12,5 ml 2,6-Dimethylmorpholin (cis/trans-Gemisch) und 0,1 g Kaliumjodid werden in 200 ml Tetrahydrofuran während 16 Std. bei Raumtemperatur gerührt. Das Reaktionsgemisch wird auf Eiswasser gegeossen und zweimal mit Ethylacetat extrahiert. Die organische Phase wird zweimal mit halbges. Natriumchlorid-Lösung gewaschen, über Natriumsulfat getrocknet, filtriert und eingeengt. Durch Chromatographie (Kieselgel, Ethylacetat/Petroleumbenzin 2 : 3) und anschliessende Kristallisation aus Ether/Petroleumbenzin werden 6,8 g der Verbindung Nr. 1.61 als cis/trans-Gemisch erhalten. Smp. 120-131°.

Beispiel H2 : Herstellung von

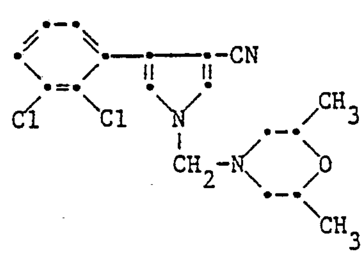

(Verb. Nr. 1.63/1.75)

N-(2,6-Dimethylmorpholin-4-ylmethyl)-3-(2,3-dichlorphenyl)-4-cyanopyrrol

Zu einer Suspension von 10 g 3-(2,3-Dichlorphenyl)-4-cyanopyrrol in 35 ml Ethanol werden 5 ml einer 38-prozentigen, wässrigen Formaldehyd-Lösung und 0,4 ml Essigsäure gegeben. Unter Rühren werden 8,3 ml 2,6-Dimethylmorpholin (cis/trans-Gemisch) zugetropft, wobei die Temperatur auf 37° steigt. Es werden anschliessend 14 ml Wasser zugetropft und die Lösung über Nacht bei Raumtemperatur stehengelassen. Anschliessend wird nochmals Wasser bis zur beginnenden Trübung zugetropft, dann im Eisbad gekühlt, wobei Kristallisation einsetzt. Die gebildeten Kristalle werden abfiltriert und über Phosphorpentoxid getrocknet. Es werden 8,5 g des gewünschten Produktes als cis/trans-Gemisch in Form brauner Kristalle erhalten Smp. 91-97°.

Die Mutterlauge wird auf ein kleines Volumen eingeengt, mit Ethylacetat und Wasser versetzt, die org. Phase abgetrennt, noch zweimal mit halbges. Natriumchlorid-Lösung gewaschen, über Natriumsulfat getrocknet und eingeengt. Der Rückstand wird aus Ether/Petroleumbenzin umkristallisiert wobei 0,8 g eines einheitlichen Diastereomeren des obigen Produktes als farblose Kristalle erhalten werden. Smp. 102-104°.

Beispiel H3 : Herstellung von

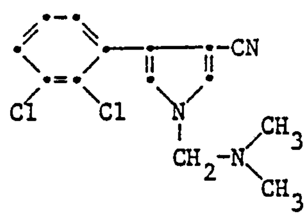

(Verb. Nr. 1.1)

N-(N',N'-Dimethylaminomethyl)-3-(2,3-dichlorphenyl)-4-cyanopyrrol

5,9 g 3-(2,3-Dichlorphenyl)-4-cyanopyrrol werden in 30 ml Ethanol suspendiert und mit 2,4 ml einer 38-prozentigen wässrigen Formaldehyd-Lösung und dann mit 3,3 ml einer 41,6-prozentigen wässrigen Dimethylamin-Lösung versetzt. Es setzt eine schwach exotherme Reaktion ein, wobei sich eine klare Lösung bildet. Nach 16 Std. bei Raumtemperatur setzt eine spontane Kristallisation ein. Es wird noch 2 Std im Kühlschrank stehen gelassen, dann die beigen Kristalle abfiltriert und getrocknet. Es werden 6,3 g des obigen Produktes erhalten. Smp. 128-130°.

Beispiel H4 : Herstellung von

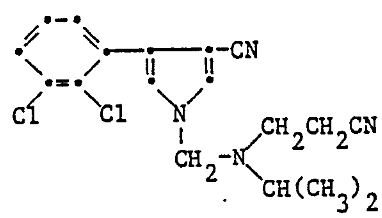

(Verb. Nr. 1.23)

N-[N'-Isopropyl-N'-(2-cyanoethyl)-aminomethyl]-3-(2,3-dichlorphenyl)-4-cyanopyrrol

Eine Suspension von 2.8 g 2-(2,3-Dichlorphenyl)-4-cyanopyrrol in 12 ml Ethanol wird mit 1 ml einer 38-prozentigen, wässrigen Formaldehyd-Lösung und dann mit 1,5 ml 3-Isopropylaminopropionitril versetzt. Das Reaktionsgemisch wird anschliessend unter Rückfluss erhitzt bis eine klare Lösung ensteht. Beim Abkühlen beginnen sich allmählich Kristalle abzuscheiden. Das Reaktionsgemisch wird noch 6 Std. bei Raumtemperatur, dann 14 Std. bei 7° stehengelassen. Anschliessend werden die farblosen Kristalle abfiltriert und getrocknet. Es werden 3 g des obigen Produktes erhalten. Smp. 104-106 °C.

Beispiel H5 : Herstellung von

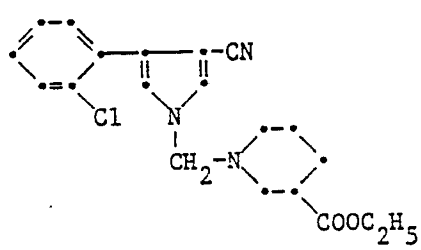

(Verb. Nr. 1.48)

N-(3-Ethoxycarbonylpiperazin-1-ylmethyl)-3-(2-chlorphenyl)-4-cyanopyrrol

4,9 g 3-(2-Chlorphenyl)-4-cyanopyrrol werden in 50 ml wasserfreiem Tetrahydrofuran gelöst, mit 0,4 ml DBU, 1,2 g Paraformaldehyd und 6,3 g Piperidin-3-carbonsäureethylester versetzt und das Gemisch über Nacht bei Raumtemperatur gerührt. Die jetzt klare Lösung wird eingeengt. Chromatographie des Rückstandes (Kieselgel, Essigsäureethylester, Petroleumbenzin) liefert obiges Produkt als gelblichen Harz. $n_D^{50} = 1,5571$.

Analog den vorstehend beschriebenen Arbeitsweisen werden auch die nachfolgend genannten Verbindungen der Formel I hergestellt :

Tabelle 1 : Verbindung der Formel

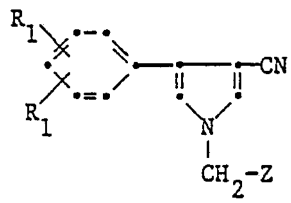

| Nr. | $R_1$ | $R_2$ | Z | Phys. Konst [°C] |
|---|---|---|---|---|
| 1.1 | 2-Cl | 3-Cl | $-N(CH_3)_2$ | Smp. 128-130° |
| 1.2 | H | H | $-N(CH_3)_2$ | Smp. 72-75° |
| 1.3 | 2-Cl | H | $-N(CH_3)_2$ | Smp. 92-96° |
| 1.4 | H | 3-Cl | 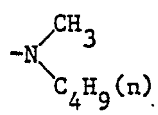 | |
| 1.5 | $2-OCH_3$ | H | 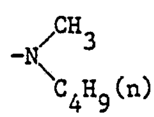 | |
| 1.6 | 2-Cl | 3-Cl | 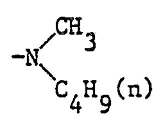 | |
| 1.7 | $2-SCH_3$ | H | $-N(C_6H_{13}(n))_2$ | |
| 1.8 | H | H | $-N(C_6H_{13}(n))_2$ | |
| 1.9 | 2-Cl | 3-Cl | 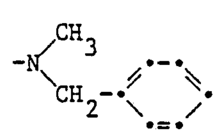 | Smp. 73-78° |
| 1.10 | H | H | 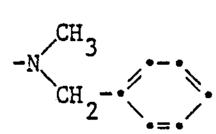 | |
| 1.11 | 2-Cl | H | 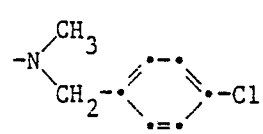 | semikristallin |

Tabelle 1 (Fortsetzung)

| Nr. | $R_1$ | $R_2$ | Z | Phys. Konst. [°C] |
|---|---|---|---|---|
| 1.12 | 2-Cl | 3-Cl | 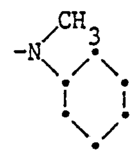 | |
| 1.13 | 2-Cl | H | 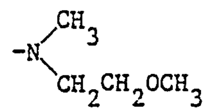 | |
| 1.14 | 2-Cl | 3-Cl | 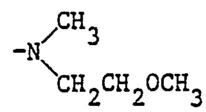 | Harz |
| 1.15 | H | H | 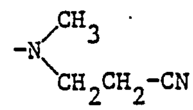 | Smp. 49-50° |
| 1.16 | 2-Cl | 3-Cl | 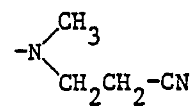 | Smp. 105-107° |
| 1.17 | H | 3-Cl | 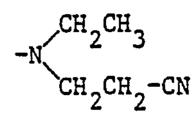 | |
| 1.18 | $2-SCH_3$ | H | 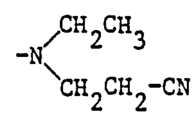 | |
| 1.19 | $2-OCH_3$ | H | 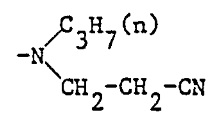 | Smp. 89-91° |
| 1.20 | 2-Cl | 3-Cl | 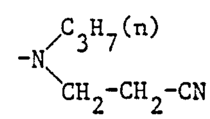 | semikristallin |
| 1.21 | 2-Cl | 3-Cl | 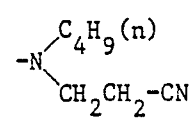 | semikristallin |
| 1. 22 | 2-Cl | 3-Cl | 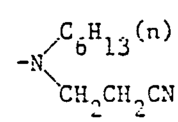 | Smp. 48-49° |

Tabelle 1 (Fortsetzung)

| Nr. | $R_1$ | $R_2$ | Z | Phys. Konst. [°C] |
|---|---|---|---|---|
| 1.23 | 2-Cl | 3-Cl | 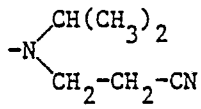 | Smp. 104-106° |
| 1.24 | 2-Cl | H | 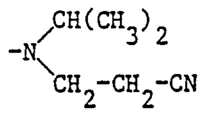 | Smp. 68-69° |
| 1.25 | 2-Cl | 3-Cl | 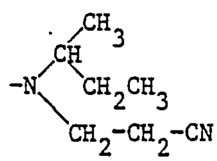 | Smp. 81-82° |
| 1.26 | 2-Cl | 3-Cl | 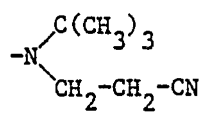 | |
| 1.27 | 2-Cl | H | 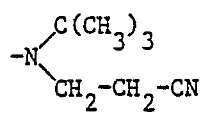 | |
| 1.28 | H | H | 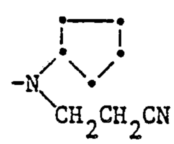 | |
| 1.29 | 2-Cl | 3-Cl | 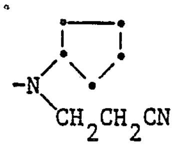 | Smp. 70-71° |
| 1.30 | 2-Cl | 3-Cl | 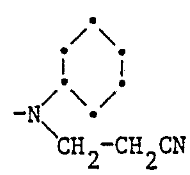 | |
| 1.31 | $2-OCH_3$ | H | 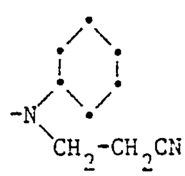 | |

Tabelle 1 (Fortsetzung)

| Nr. | $R_1$ | $R_2$ | Z | Phys. Konst. [°C] |
|---|---|---|---|---|
| 1.32 | 2-Cl | 3-Cl | 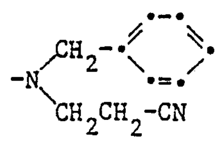 | Smp. 104-106° |
| 1.33 | H | H | 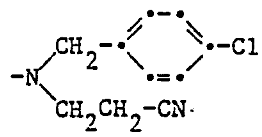 | Smp. 89-91° |
| 1.34 | 2-Cl | 3-Cl | 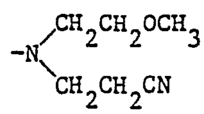 | Smp. 73-75° |
| 1.35 | 2-Cl | H | 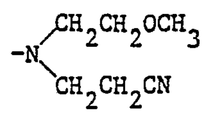 | Smp. 50-52° |
| 1.36 | 2-Cl | 3-Cl | 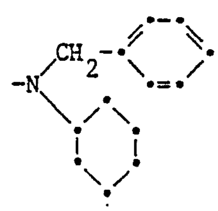 | Harz |
| 1.37 | 2-Cl | 3-Cl | 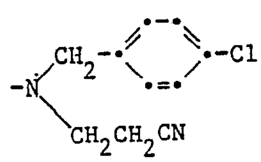 | Smp. 119-120° |
| 1.38 | H | 3-Cl | 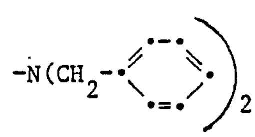 | |
| 1.39 | H | H | 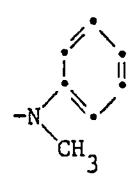 | |

Tabelle 1 (Fortsetzung)

| Nr. | $R_1$ | $R_2$ | Z | Phys. Konst. [°C] |
|---|---|---|---|---|
| 1.40 | 2-Cl | 3-Cl | 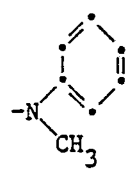 | |
| 1.41 | 2-Cl | H | 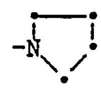 | |
| 1.42 | 2-Cl | 3-Cl | 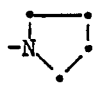 | Smp. 57-59° |
| 1.43 | 2-Cl | 3-Cl | 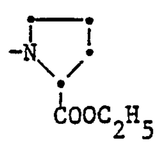 | |
| 1.44 | H | H | 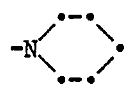 | |
| 1.45 | 2-Cl | 3-Cl | 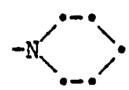 | Smp. 94-96° |
| 1.46 | 2-Cl | H | 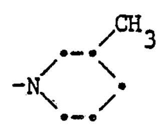 | |
| 1.47 | 2-Cl | 3-Cl | 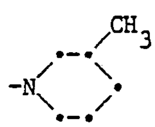 | |
| 1.48 | 2-Cl | H | 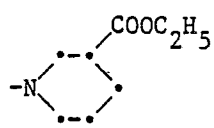 | $n_D^{50}$ : 1,5571 |

Tabelle 1 (Fortsetzung)

| Nr. | $R_1$ | $R_2$ | Z | Phys. Konst. [°C] |
|---|---|---|---|---|
| 1.49 | 2-Cl | 3-Cl | 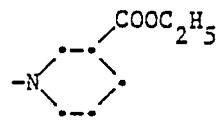 | |
| 1.50 | 2-$OCH_3$ | H | 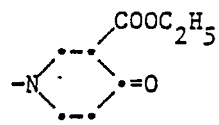 | Harz |
| 1.51 | H | H | 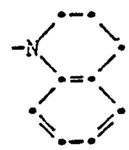 | |
| 1.52 | 2-$SCH_3$ | H | 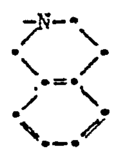 | |
| 1.53 | H | 3-Cl | 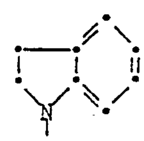 | |
| 1.54 | H | 3-Cl | 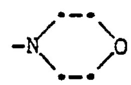 | |
| 1.55 | 2-Cl | 3-Cl | 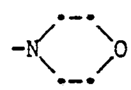 | Smp. 95-97° |
| 1.56 | 2-Cl | H | 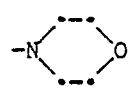 | Smp. 81-82° |
| 1.57 | H | H | 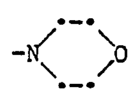 | |

Tabelle 1 (Fortsetzung)

| Nr. | $R_1$ | $R_2$ | Z | Phys. Konst.[°C] |
|---|---|---|---|---|
| 1.58 | 2-Cl | 3-Cl | 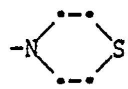 | |
| 1.59 | H | H | 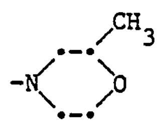 | |
| 1.60 | 2-Cl | 3-Cl | 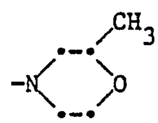 | |
| 1.61 | 2-Cl | H | 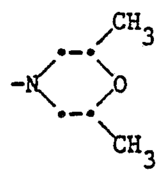 1) | Smp. 120-131° |
| 1.62 | H | 3-Cl | 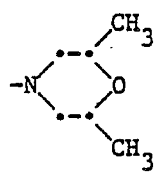 1) | Oel |
| 1.63 | 2-Cl | 3-Cl | 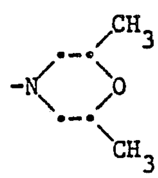 1) | Smp. 91-97° |
| 1.64 | $2-SCH_3$ | H | 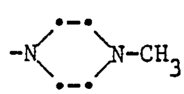 | |
| 1.65 | 2-Cl | H | 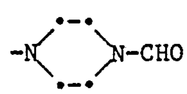 | |
| 1.66 | 2-Cl | 3-Cl | 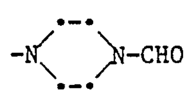 | |

Tabelle 1 (Fortsetzung)

| Nr. | $R_1$ | $R_2$ | Z | Phys. Konst.[°C] |
|---|---|---|---|---|
| 1.67 | H | H | 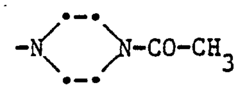 | Semikristallin |
| 1.68 | 2-Cl | 3-Cl | 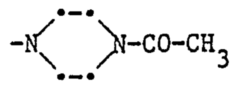 | Smp. 170-173 |
| 1.69 | H | 3-Cl | 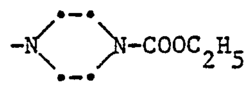 | |
| 1.70 | 2-Cl | 3-Cl | 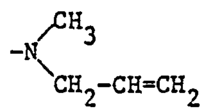 | Harz |
| 1.71 | H | H | $-N(CH_2-CH=CH_2)_2$ | |
| 1.72 | 2-Cl | 3-Cl | 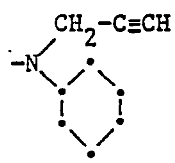 | Harz |
| 1.73 | 2-Cl | 3-Cl | 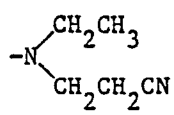 | |
| 1.74 | 2-Cl | 3-Cl | 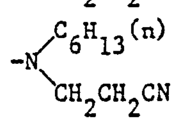 | |
| 1.75 | 2-Cl | 3-Cl | 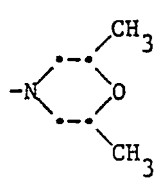 2) | Smp. 102-104° |
| 1.76 | 2-Cl | 3-Cl | 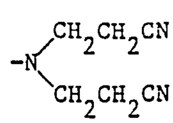 | Smp. 142-144° |
| 1.77 | 2-Cl | 3-Cl | 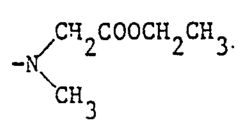 | |

1) Gemische von cis + trans Isomeren
2) Reines Isomeres

Formulierungsbeispiele für flüssige Wirkstoffe der Formel I (% = Gewichtsprozent)

| F1. Emulsions-Konzentrate | a) | b) | c) |
|---|---|---|---|
| Wirkstoff aus den Tabellen | 25% | 40% | 50% |
| Ca-Dodecylbenzolsulfonat | 5% | 8% | 6% |
| Ricinusöl-polyethylenglykolether (36 Mol Ethylenoxid) | 5% | - | - |
| Tributylphenoyl-polyethylenglykolether (30 Mol Ethylenoxid) | - | 12% | 4% |
| Cyclohexanon | - | 15% | 20% |
| Xylolgemisch | 65% | 25% | 20% |

Aus solchen Konzentraten können durch Verdünnen mit Wasser Emulsionen jeder gewünschten Konzentration hergestellt werden.

| F2. Lösungen | a) | b) | c) | d) |
|---|---|---|---|---|
| Wirkstoff aus den Tabellen | 80% | 10% | 5% | 95% |
| Ethylenglykol-monomethyl-ether | 20% | - | - | - |
| Polyethylenglykol M G 400 | - | 70% | - | - |
| N-Methyl-2-pyrrolidon | - | 20% | - | - |
| Epoxydiertes Kokosnussöl | - | - | 1% | 5% |
| Benzin (Siedegrenzen 160-190°C) | - | - | 94% | - |

(MG = Molekulargewicht)

| F3. Granulate | a) | b) |
|---|---|---|
| Wirkstoff aus den Tabellen | 5% | 10% |
| Kaolin | 94% | - |
| Hochdisperse Kieselsäure | 1% | - |
| Attapulgit | - | 90% |

Der Wirkstoff wird in Methylenchlorid gelöst, auf den Träger aufgesprüht und das Lösungsmittel anschliessend im Vakuum abgedampft.

| F4. Stäubemittel | a) | b) |
|---|---|---|
| Wirkstoff aus den Tabellen | 2% | 5% |
| Hochdisperse Kieselsäure | 1% | 5% |
| Talkum | 97% | - |
| Kaolin | - | 90% |

Durch inniges Vermischen auf Trägerstoffe mit dem Wirkstoff erhält man gebrauchsfertige Stäubemittel.

Formulierungsbeispiele für feste Wirkstoffe der Formel I (% = Gewichtsprozent)

| F5. Spritzpulver | a) | b) | c) |
|---|---|---|---|
| Wirkstoff aus den Tabellen | 25% | 50% | 75% |
| Na-Ligninsulfonat | 5% | 5% | - |
| Na-Laurylsulfat | 3% | - | 5% |
| Na-Diisobutylnaphthalinsulfonat | - | 6% | 10% |
| Octylphenolpolyethylenglykolether (7-8 Mol Ethylenoxid) | - | 2% | - |
| Hochdisperse Kieselsäure | 5% | 10% | 10% |
| Kaolin | 62% | 27% | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| F6. Emulsions-Konzentrat | |
|---|---|
| Wirkstoff aus den Tabellen | 10% |
| Octylphenolpolyethylenglykolether (4-5 Mol Ethylenoxid) | 3% |
| Ca-Dodecylbenzolsulfonat | 3% |
| Ricinusölpolyglykolether (35 Mol Ethylenoxid) | 4% |
| Cyclohexanon | 30% |
| Xylolgemisch | 50% |

Aus diesem Konzentrat können durch Verdünnen mit Wasser Emulsionen jeder gewünschten Konzentration hergestellt werden.

| F7. Stäubemittel | a) | b) |
|---|---|---|
| Wirkstoff aus den Tabellen | 5% | 8% |
| Talkum | 95% | - |
| Kaolin | - | 92% |

Man erhält anwendungsfertige Stäubemittel, indem der Wirkstoff mit den Träger vermischt auf einer geeigneten Mühle vermahlen wird.

| F8. Extruder Granulat | |
|---|---|
| Wirkstoff aus den Tabellen | 10% |
| Na-Ligninsulfonat | 2% |
| Carboxymethylcellulose | 1% |
| Kaolin | 87% |

Der Wirkstoff wird mit den Zusatzstoffen vermischt, vermahlen und mit Wasser angefeuchtet. Dieses Gemisch wird extrudiert und anschliessend im Luftstrom getrocknet.

F9. Umhüllungs-Granulat

| | |
|---|---|
| Wirkstoff aus den Tabellen | 3% |
| Polyethylenglykol (M G 200) | 3% |
| Kaolin | 94% |

(MG = Molekulargewicht)

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

F10. Suspensions-Konzentrat

| | |
|---|---|
| Wirkstoff aus den Tabellen | 40% |
| Ethylenglykol | 10% |
| Nonylphenolpolyethylenglykolether (15 Mol Ethylenoxid) | 6% |
| N-Ligninsulfonat | 10% |
| Carboxymethylcellulose | 1% |
| 37%ige wässrige Formaldehyd-Lösung | 0,2% |
| Silikonöl in Form einer 75%igen wässrigen Emulsion | 0,8% |
| Wasser | 32% |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

## Biologische Beispiele

Beispiel B1 : Wirkung gegen Puccinia graminis auf Weizen

a) Residual-protektive Wirkung

Weizenpflanzen wurden 6 Tage nach der Aussaat mit einer aus Spritzpulver des Wirkstoffes hergestellten Spritzbrühe (0,06 % Aktivsubstanz) besprüht. Nach 24 Stunden wurden die behandelten Pflanzen mit einer Uredosporensuspension des Pilzes infiziert. Nach einer Inkubation während 48 Stunden bei 95-100 % relativer Luftfeuchtigkeit und ca. 20 °C wurden die infizierten Pflanzen in einem Gewächshaus bei ca. 22 °C aufgestellt. Die Beurteilung der Rostpustelnentwicklung erfolgte 12 Tage nach der Infektion.

b) Systemische Wirkung

Zu Weizenpflanzen wurde 5 Tage nach der Aussaat eine aus Spritzpulver des Wirkstoffes hergestellte Spritzbrühe gegossen (0,006 % Aktivsubstanz bezogen auf das Bodenvolumen). Nach 48 Stunden wurden die behandelten Pflanzen mit einer Uredosporensuspension des Pilzes infiziert. Nach einer Inkubation während 48 Stunden bei 95-100 % relativer Luftfeuchtigkeit und ca. 20 °C wurden die infizierten Pflanzen in einem Gewächshaus bei ca. 22 °C aufgestellt. Die Beurteilung der Rostpustelnentwicklung erfolgte 12 Tage nach der Infektion.

Verbindungen aus den Tabellen zeigten nicht nur im Gewächshaus sondern auch im Freiland gegen Puccinia-Pilze eine gute Wirkung. Unbehandelte aber infizierte Kontrollpflanzen zeigten einen Puccinia-Befall von 100 %. Unter anderen hemmten die Verbindungen Nr. 1.11, 1.14, 1.15, 1.20, 1.21, 1.22, 1.23, 1.24, 1.28, 1.29, 1.33, 1.36, 1.37, 1.50 und 1.72 den Puccinia-Befall auf 0 bis 10 %.

Beispiel B2 : Wirkung gegen Cercospora arachidicola auf Erdnusspflanzen

a) Residual-protektive Wirkung

10-15 cm hohe Erdnusspflanzen wurden mit einer aus Spritzpulver der Wirksubstanz hergestellten Spritzbrühe (0,006 Aktivsubstanz) besprüht und 48 Stunden später mit einer Konidiensuspension des

Pilzes infiziert. Die infizierten Pflanzen wurden während 72 Stunden bei ca. 21 °C und hoher Luftfeuchtigkeit inkubiert und anschliessend bis zum Auftreten der typischen Blattflecken in einem Gewächshaus aufgestellt. Die Beurteilung der fungiziden Wirkung erfolgt 12 Tage nach der Infektion basierend auf Anzahl und Grösse der auftretenden Flecken.

b) Systemische Wirkung

Zu 10-15 cm hohen Erdnusspflanzen wurde eine aus Spritzpulver des Wirkstoffes hergestellte Spritzbrühe gegossen (0,06 % Aktivsubstanz bezogen auf das Erdvolumen). Nach 48 Stunden wurden die behandelten Pflanzen mit einer Konidiensuspension des Pilzes infiziert und 72 Stunden bei ca. 21 °C und hoher Luftfeuchtigkeit inkubiert. Anschliessend wurden die Pflanzen im Gewächshaus aufgestellt und nach 11 Tagen der Pilzbefall beurteilt.

Im Vergleich zu unbehandelten, aber infizierten Kontrollpflanzen (Anzahl und Grösse der Flecken = 100 %), zeigten Erdnusspflanzen, die im Gewächshaus oder im Freiland mit Wirkstoffen aus den Tabellen behandelt wurden, einen stark reduzierten Cercospora-Befall. So verhinderten die Verbindungen Nr. 1.9, 1.20, 1.21, 1.22, 1.23, 1.45, 1.55, 1.56, 1.63 und 1.68 in obigen Versuchen das Auftreten von Flecken fast vollständig (8 %).

Beispiel B3 : Wirkung gegen Botrytis cinerea auf Bohnen

Residual protektive Wirkung

Ca. 10 cm hohe Bohnen-Pflanzen wurden mit einer aus Spritzpulver des Wirkstoffes hergestellten pritzbrühe (0,02 % Aktivsubstanz) besprüht. Nach 48 Stunden wurden die behandelten Pflanzen mit einer Konidiensuspension des Pilzes infiziert. Nach einer Inkubation der infizierten Pflanzen während 3 Tagen bei 95-100 % relativer Luftfeuchtigkeit und 21 °C erfolgte die Beurteilung des Pilzbefalls. Die Verbindungen aus den Tabellen hemmten nicht nur im obigen Modellversuch sondern auch im Freiland die Pilzinfektion sehr stark. Bei einer Konzentration von 0,02 % erwiesen sich z. B. die Verbindungen Nr. 1.1, 1.2, 1.3, 1.9, 1.11, 1.14, 1.15, 1.16, 1.19 bis 1.25, 1.29, 1.32 bis 1.37, 1.42, 1.45, 1.48, 1.50, 1.55, 1.56, 1.61, 1.63, 1.68, 1.70, 1.72, 1.75 und 1.76 als voll wirksam (Krankheitsbefall 0 bis 5 %). Befall unbehandelter aber infizierter Bohnenpflanzen betrug 100 %. Gleich gut wirken auch die Zwischenprodukte Nr. 16 und 24.

Beispiel B4 : Wirkung gegen Botrytis cinerea an Apfelfrüchten

Künstlich verletzte Aepfel wurden behandelt, indem eine aus Spritzpulver der Wirksubstanz hergestellte Spritzbrühe auf die Verletzungsstellen aufgetropft wurde. Die behandelten Früchte wurden anschliessend mit einer Sporensuspension von Botrytis cinerea inokuliert und während einer Woche bei einer hohen Luftfeuchtigkeit und ca. 20 °C inkubiert.

Bei der Auswertung wurden die angefaulten Verletzungsstellen gezählt und daraus die fungizide Wirkung der Testsubstanz abgeleitet. Unter anderen hemmten die Verbindungen Nr. 1.1, 1.2, 1.3, 1.9, 1.11, 1.14, 1.15, 1.16, 1.19 bis 1.25, 1.29, 1.32 bis 1.37, 1.42, 1.45, 1.48, 1.50, 1.55, 1.56, 1.61, 1.62, 1.63, 1.68, 1.70, 1.72, 1.75 und 1.76 den Pilzbefall im Vergleich zu unbehandelten Kontrollfrüchten (100 % Befall) fast vollständig. Gleich gut wirken auch die Zwischenprodukte Nr. 16 und 24.

Beispiel B5 : Wirkung gegen Piricularia oryzae auf Reis

Reispflanzen wurden nach zweiwöchiger Anzucht mit einer aus Spritzpulver des Wirkstoffes hergestellten Spritzbrühe (0,02 % Aktivsubstanz) besprüht. Nach 48 Stunden wurden die behandelten Pflanzen mit einer Konidiensuspension des Pilzes infiziert. Nach 5 Tagen Inkubation bei 95-100 % relativer Luftfeuchtigkeit und 24 °C wurde der Pilzbefall beurteilt. Verbindungen der Formel I zeigten eine gute Hemmung des Piriculariabefalls, so z. B. die Verbindungen Nr. 1.1, 1.16, 1.55, 1.61, 1.62 und 1.63 ; sie reduzierten den Befall auf weniger als 10 %.

Beispiel B6 : Wirkung gegen Rhizoctonia solani auf Kohl

Wirkung nach Bodenapplikation

Der Pilz wurde auf sterilen Hirsekörnern kultiviert und einer Erde-Sand-Mischung beigegeben. Die infizierte Erde wurde in Schalen abgefüllt und mit Kohlsamen besät. Gleich nach der Aussaat wurde das als Spritzpulver formulierte Versuchspräparat als wässrige Suspension über die Erde gegossen (20 ppm Aktivsubstanz bezogen auf das Erdvolumen). Die Erdschalen wurden darauf 2-3 Wochen bei ca. 24 °C im Gewächshaus aufgestellt und durch leichtes Ueberbrausen stets gleichmässig feucht gehalten. Bei der Auswertung des Tests wurde der Auflauf der Kohlpflanzen bestimmt.

Nach Behandlung mit Spritzpulvern, die als Aktivsubstanz eine der Verbindungen Nr. 1.1, 1.61, 1.62 oder 1.63 enthielten, liefen über 80 % der Kohlsamen auf, und die Pflanzen hatten ein gesundes Aussehen.

Beispiel B7 : Residual protective Wirkung gegen Venturia inaequalis auf Apfeltrieben

Apfelstecklinge mit 10-20 cm langen Frischtrieben wurden mit einer aus Spritzpulver des Wirkstoffes hergestellten Spritzbrühe (0,06 % Aktivsubstanz) besprüht. Nach 24 Stunden wurden die behandelten Pflanzen mit einer Konidiensuspension des Pilzes infiziert. Die Pflanzen wurden dann während 5 Tagen bei 90-100 % relativer Luftfeuchtigkeit inkubiert und während 10 weiteren Tagen in einem Gewächshaus bei 20-24 °C aufgestellt. Der Schorfbefall wurde 15 Tage nach der Infektion beurteilt. Verbindungen der Formel I zeigten eine gute Wirkung gegen Venturia-Erreger, insbesondere die Verbindungen Nr. 1.1, 1.9, 1.45, 1.55, 1.62 und 1.63 hemmten den Schorbefall im Vergleich zu unbehandelten Kontrollpflanzen auf weniger als 20 %.

Beispiel B8 : Wirkung gegen Helminthosporium gramineum

Weizenkörner werden mit einer Sporensuspension des Pilzes kontaminiert und wieder angetrocknet. Die kontaminierten Körner werden mit einer aus Spritzpulver hergestellten Suspension der Testsubstanz gebeizt (600 ppm Wirkstoff bezogen auf das Gewicht der Samen). Nach zwei Tagen werden die Körner auf geeignete Agarschalen ausgelegt und nach weiteren vier Tagen wird die Entwicklung der Pilzkolonien um die Körner herum beurteilt. Anzahl und Grösse der Pilzkolonien werden zur Beurteilung der Testprodukte herangezogen. Dabei verhinderten die Verbindungen aus den Tabellen den Pilzbefall weitgehend (0 bis 10 %).

Beispiel B9 : Wirkung gegen Fusarium nivale

Weizenkörner werden mit einer Sporensuspension des Pilzes kontaminiert und wieder angetrocknet. Die kontaminierten Körner werden mit einer aus Spritzpulver hergestellten Suspension der Testsubstanz gebeizt (600 ppm Wirkstoff bezogen auf das Gewicht der Samen). Nach zwei Tagen werden die Körner auf geeignete Agarschalen ausgelegt und nach weiteren vier Tagen wird die Entwicklung der Pilzkolonien um die Körner herum beurteilt. Anzahl und Grösse der Pilzkolonien werden zur Beurteilung der Testprodukte herangezogen.

Bei den Körnern, die mit einem Spritzpulver behandelt worden sind, das als Wirkstoff eine der Verbindungen aus den Tabellen enthielt, wurde die Entwicklung der Pilzkolonien fast vollständig unterdrückt (0 bis 5 %).

Beispiel B10 : Wirkung gegen Alternaria solani auf Tomate

Tomatenpflanzen wurden nach dreiwöchiger Anzucht mit einer aus Spritzpulver des Wirkstoffs hergestellten Spritzbrühe (0,006 % Aktivsubstanz) besprüht. Nach 24 Stunden wurden die Tomatenpflanzen mit einer Konidiensuspension des Pilzes infiziert. Die Beurteilung der fungiziden Wirkung erfolgte aufgrund des Pilzbefalls nach einer Inkubation der infizierten Pflanzen während 8 Tagen bei hoher Luftfeuchtigkeit und einer Temperatur von 18-22°.

Verbindungen aus den Tabellen zeigten nicht nur in obigem Modellversuch, sondern auch unter Freilandbedingungen eine sehr gute Wirkung gegen Alternaria solani. So reduzierten unter anderen die Verbindungen Nr. 1.1, 1.2, 1.3, 1.9, 1.15, 1.16, 1.19, 1.22, 1.23, 1.24, 1.25, 1.29, 1.34, 1.35, 1.45, 1.48, 1.55, 1.56, 1.61, 1.62, 1.63 und 1.68 den Pilzbefall auf 0 bis 10 %, während die unbehandelten aber infizierten Kontrollpflanzen einen Pilzbefall von 100 % aufwiesen.

**Patentansprüche** (für die Vertragsstaaten : GB, BE, DE, FR, IT, LU, NL, SE, CH, LI)

1. Verbindungen der Formel I

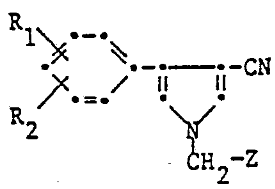

(I)

worin

$R_1$ und $R_2$ unabhängig voneinander für Wasserstoff, Halogen, Methoxy oder Methylthio stehen ;

Z für eine der Gruppen

a) 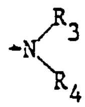 oder

b) 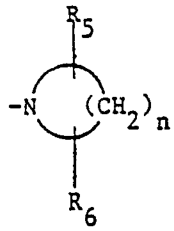 oder

c) 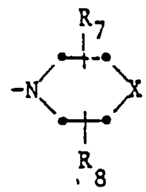

steht, wobei

$R_3$ und $R_4$ unabhängig voneinander für Wasserstoff, unsubstituiertes oder durch Cyano, $C_1$-$C_6$-Alkoxy oder $C_1$-$C_6$-Alkoxycarbonyl substituiertes $C_1$-$C_6$-Alkyl, $C_3$-$C_6$-Alkenyl, $C_3$-$C_6$-Alkinyl, $C_3$-$C_7$-Cycloalkyl, unsubstituiertes oder durch Halogen, $C_1$-$C_6$-Alkyl, $C_1$-$C_6$-Haloalkyl und/oder $C_1$-$C_6$-Alkoxy substituiertes Benzyl oder unsubstituiertes oder durch Halogen, $C_1$-$C_6$-Alkyl, $C_1$-$C_6$-Haloalkyl und/oder $C_1$-$C_6$-Alkoxy substituiertes Phenyl stehen, mit der Massgabe, dass nur $R_3$ oder $R_4$ Wasserstoff sein kann ;

$R_5$ und $R_6$ unabhängig voneinander für Wasserstoff, $C_1$-$C_6$-Alkyl, $C_1$-$C_6$-Alkoxycarbonyl stehen oder beide zusammen einen ankondensierten Phenylring bilden ;

n für eine der Zahlen 4 oder 5 steht ;

X Sauerstoff, Schwefel, $>C{=}O$ oder $>N{-}R_9$ bedeutet ;

$R_7$ und $R_8$ unabhängig voneinander für Wasserstoff, $C_1$-$C_6$-Alkyl oder $C_1$-$C_6$-Alkoxycarbonyl stehen ; und

$R_9$ Wasserstoff, $C_1$-$C_6$-Alkyl, Formyl ; $C_1$-$C_6$-Alkanoyl oder $C_1$-$C_6$-Alkoxycarbonyl bedeutet.

2. Verbindungen der Formel I nach Anspruch 1, worin $R_1$ und $R_2$ unabhängig voneinander für Wasserstoff, Chlor, Methoxy oder Methylthio stehen ; Z für eine der Gruppen

a) 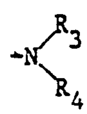 oder

b) 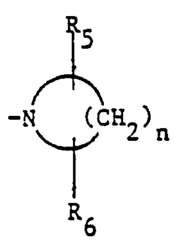 oder

c) 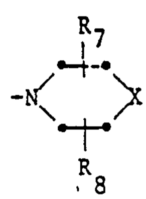

steht, wobei $R_3$ und $R_4$ unabhängig voneinander für Wasserstoff, $C_1$-$C_6$-Alkyl, durch Cyano, $C_1$-$C_3$-Alkoxy oder $C_1$-$C_3$-Alkoxycarbonyl substituiertes $C_1$-$C_3$-Alkyl, $C_3$-$C_4$-Alkenyl, $C_3$-$C_4$-Alkinyl, $C_5$-$C_6$-

Cycloalkyl, unsubstituiertes oder durch Halogen, Methyl, Trifluormethyl und/oder Methoxy substituiertes Benzyl oder unsubstituiertes oder durch Halogen, Methyl, Trifluormethyl und/oder Methoxy substituiertes Phenyl stehen, mit der Massgabe, dass nur $R_3$ oder $R_4$ Wasserstoff sein kann ; $R_5$ und $R_6$ unabhängig voneinander für Wasserstoff, $C_1$-$C_3$-Alkyl oder $C_1$-$C_3$-Alkoxycarbonyl stehen oder beide zusammen einen ankondensierten Phenylring bilden ; n für eine der Zahlen 4 oder 5 steht ; X Sauerstoff, Schwefel, >C=O oder >N—$R_9$ bedeutet ; $R_7$ und $R_8$ unabhängig voneinander für Wasserstoff, $C_1$-$C_3$-Alkyl oder $C_1$-$C_3$-Alkoxycarbonyl stehen ; und $R_9$ Wasserstoff, $C_1$-$C_3$-Alkyl, Formyl, $C_1$-$C_3$-Alkanoyl oder $C_1$-$C_3$-Alkoxycarbonyl bedeutet.

3. Verbindungen der Formel I nach Anspruch 2, worin $R_1$ in der 2-Position und $R_2$ in der 3-Position fixiert sind und unabhängig voneinander für Wasserstoff, Chlor, Methoxy oder Methylthio stehen ; Z für eine der Gruppen

a) 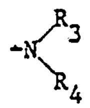 oder

b) 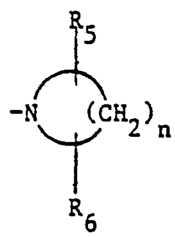 oder

c) 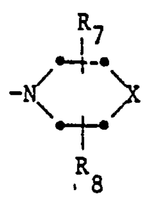

steht, wobei $R_3$ und $R_4$ unabhängig voneinander für $C_1$-$C_6$-Alkyl, durch Cyano, $C_1$-$C_3$-Alkoxy oder $C_1$-$C_3$-Alkoxycarbonyl substituiertes $C_1$-$C_2$-Alkyl, Allyl, Propargyl, Cyclopentyl, Cyclohexyl, Benzyl, Halobenzyl, Phenyl oder Halophenyl stehen ; $R_5$ und $R_6$ unabhängig voneinander für Wasserstoff, Methyl oder $C_1$-$C_3$-Alkoxycarbonyl stehen oder beide zusammen einen ankondensierten Phenylring bilden ; n für eine der Zahlen 4 oder 5 steht ; X Sauerstoff, Schwefel, >C=O oder >R—$N_9$ bedeutet ; $R_7$ und $R_8$ unabhängig voneinander für Wasserstoff, Methyl oder $C_1$-$C_3$-Alkoxycarbonyl stehen ; und $R_9$ Wasserstoff, $C_1$-$C_2$-Alkyl, Formyl, $C_1$-$C_3$-Alkanoyl oder $C_1$-$C_3$-Alkoxycarbonyl bedeutet.

4. Verbindungen der Formel I nach Anspruch 3, worin $R_1$ für Wasserstoff oder 2-Chlor und $R_2$ für Wasserstoff oder 3-Chlor stehen ; Z für eine der Gruppen

a) 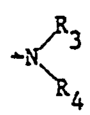 oder

b) 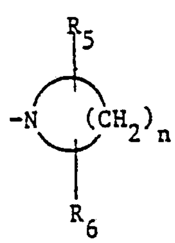 oder

c) 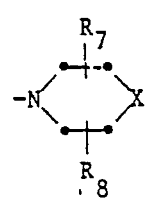

steht, wobei $R_3$ und $R_4$ unabhängig voneinander für $C_1$-$C_6$-Alkyl, durch Cyano, $C_1$-$C_3$-Alkoxy oder $C_1$-$C_3$-Alkoxycarbonyl substituiertes $C_1$-$C_2$-Alkyl, Allyl, Propargyl, Cyclopentyl, Cyclohexyl, Benzyl, Halobenzyl, Phenyl oder Halophenyl stehen; $R_5$ und $R_6$ unabhängig voneinander für Wasserstoff, Methyl oder $C_1$-$C_3$-Alkoxycarbonyl stehen oder beide zusammen einen ankondensierten Phenylring bilden; n für eine der Zahlen 4 oder 5 steht; X Sauerstoff, Schwefel, $>C{=}O$ oder $>N{-}R_9$ bedeutet; $R_7$ und $R_8$ unabhängig voneinander für Wasserstoff, Methyl oder $C_1$-$C_3$-Alkoxycarbonyl stehen; und $R_9$ Wasserstoff, $C_1$-$C_2$-Alkyl, Formyl, $C_1$-$C_3$-Alkanoyl oder $C_1$-$C_3$-Alkoxycarbonyl bedeutet.

5. Eine Verbindung der Formel I nach Anspruch 1, ausgewählt aus der Reihe
N-(N',N'-Dimethylaminomethyl)-3-(2,3-dichlorphenyl)-4-cyanopyrrol,
N-(N'-Benzyl-N'-methylaminomethyl)-3-(2,3-dichlorphenyl)-4-cyanopyrrol,
N-[N'-Methyl-N'-(2-cyanoethyl)-aminomethyl]-3-(2,3-dichlorphenyl)-4-cyanopyrrol,
N-[N'-Isopropyl-N'-(2-cyanoethyl)-aminomethyl]-3-(2,3-dichlorphenyl)-4-cyanopyrrol,
N-(Piperidin-1-ylmethyl)-3-(2,3-dichlorphenyl)-4-cyanopyrrol,
N-(3-Ethoxycarbonylpiperidin-1-ylmethyl)-3-(2-chlorphenyl)-4-cyanopyrrol,
N-(Morpholin-1-ylmethyl)-3-(2,3-dichlorphenyl)-4-cyanopyrrol,
N-(2,6-Dimethylmorpholin-1-ylmethyl)-3-(2-chlorphenyl)-4-cyanopyrrol,
N-[N'-Cyclopentyl-N'-(2-cyanoethyl)-aminomethyl]-3-(2,3-dichlorphenyl)-4-cyanopyrrol,
N-(2,6-Dimethylmorpholin-1-ylmethyl)-3-(2,3-dichlorphenyl)-4-cyanopyrrol,
N-(4-Acetylcarbonylpiperazin-1-ylmethyl)-3-(2,3-dichlorphenyl)-4-cyanopyrrol,
N-[N'-(2-Methoxyethyl)-N'-(2-cyanoethyl)-aminomethyl]-3-(2,3-dichlorphenyl)-4-cyanopyrrol,
N-[N'-Methyl-N'-(2-cyanoethyl)-aminomethyl]-3-(2-chlorphenyl)-4-cyanopyrrol,
N-[N'-Methyl-N'-(2-methoxyethyl)-aminomethyl]-3-(2,3-dichlorphenyl)-4-cyanopyrrol,
N-[N'-(n-Propyl)-N'-(2-cyanoethyl)-aminomethyl]-3-(2,3-dichlorphenyl)-4-cyanopyrrol,
N-[N'-(n-Butyl)-N'-(2-cyanoethyl)-aminomethyl]-3-(2,3-dichlorphenyl)-4-cyanopyrrol,
N-[N'-Methyl-N'-prop-2-enyl-aminomethyl]-3-(2,3-dichlorphenyl)-4-cyanopyrrol,
N-[N'-Propargyl-N'-cyclohexyl-aminomethyl]-3-(2,3-dichlorphenyl)-4-cyanopyrrol.

6. Verfahren zur Herstellung der in Anspruch 1 definierten Verbindungen der Formel I, dadurch gekennzeichnet, dass man entweder

A) eine Verbindung der Formel II

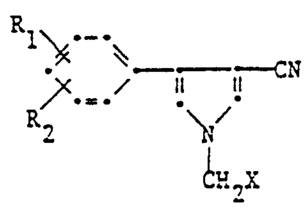

(II)

worin $R_1$ und $R_2$ die unter Formel I angegebenen Bedeutungen haben und X für OH oder Halogen steht, in Gegenwart einer Base und gegebenenfalls im Beisein eines Reaktionskatalysators bei Temperaturen von 0° bis 100 °C mit einer Verbindung der Formel III

$$H{-}Z \qquad (III)$$

umsetzt, worin Z die unter Formel I angegebenen Bedeutungen hat; oder

B) eine Verbindung der Formel IV

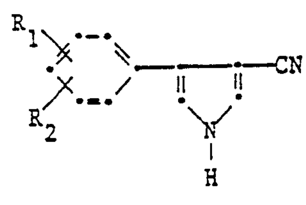

(IV)

worin $R_1$ und $R_2$ die unter Formel I angegebenen Bedeutungen haben, entweder in einem protischen Lösungsmittel bei Temperaturen von 0° bis 120 °C in Gegenwart eines basischen oder sauren Reaktionskatalysators mit Formaldehyd und einer Verbindung der Formel III umsetzt oder in einem aprotischen Lösungsmittel im Beisein eines basischen Reaktionskatalysators und bei Temperaturen von 0° bis 120 °C mit 1,3,5-Trioxan oder Paraformaldehyd umsetzt.

7. Mikrobizides Mittel zur Bekämpfung oder Verhütung eines Befalls von lebenden Pflanzen und/oder zur Konservierung von verderblichem Lagergut pflanzlicher oder tierischer Herkunft, dadurch gekennzeichnet, dass es als mindestens eine aktive Komponente eine der in Anspruch 1 definierten Verbindungen enthält.

8. Mittel gemäss Anspruch 7, dadurch gekennzeichnet, dass es als aktive Komponente mindestens eine der in den Ansprüchen 2 bis 5 definierten Verbindungen enthält.

9. Verfahren zur Bekämpfung oder Verhütung eines Befalls von Kulturpflanzen durch phytopathogene Mikroorganismen, dadurch gekennzeichnet, dass man eine Verbindung der Formel I gemäss einem der Ansprüche 1 bis 5 auf die Pflanze, Pflanzenteile oder deren Standort appliziert.

10. Verfahren zur Konservierung bzw. zum Schutz von Vorrats- oder Lagergut pflanzlicher und/oder tierischer Herkunft vor schädlichen Mikroorganismen durch Behandlung des Gutes mit mindestens einer mikrobizid wirksamen Menge einer Verbindung der Formel I gemäss einem der Ansprüche 1 bis 5.

11. Verwendung von Verbindungen der Formel I gemäss Anspruch 1 zur Bekämpfung und/oder zur präventiven Verhütung eines Befalls von Mikroorganismen.

12. Verwendung gemäss Anspruch 11, dadurch gekennzeichnet, dass es sich bei den Mikroorganismen und phytopathogene Pilze handelt.

**Patentansprüche** (für den Vertragsstaat AT)

1. Verfahren zur Herstellung einer Verbindung der Formel I

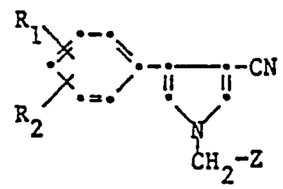

(I)

worin

$R_1$ und $R_2$ unabhängig voneinander für Wasserstoff, Halogen, Methoxy oder Methylthio stehen ;

Z für eine der Gruppen

a) 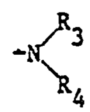 oder

b) 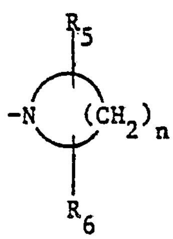 oder

c) 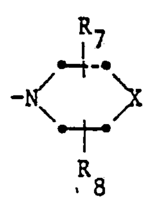

steht, wobei

$R_3$ und $R_4$ unabhängig voneinander für Wasserstoff, unsubstituiertes oder durch Cyano, $C_1$-$C_6$-Alkoxy oder $C_1$-$C_6$-Alkoxycarbonyl substituiertes $C_1$-$C_6$-Alkyl, $C_3$-$C_6$-Alkenyl, $C_3$-$C_6$-Alkinyl, $C_3$-$C_7$-Cycloalkyl, unsubstituiertes oder durch Halogen, $C_1$-$C_6$-Alkyl, $C_1$-$C_6$-Haloalkyl und/oder $C_1$-$C_6$-Alkoxy substituiertes Benzyl oder unsubstituiertes oder durch Halogen, $C_1$-$C_6$-Alkyl, $C_1$-$C_6$-Haloalkyl und/oder $C_1$-$C_6$-Alkoxy substituiertes Phenyl stehen, mit der Massgabe, dass nur $R_3$ oder $R_4$ Wasserstoff sein kann ;

$R_5$ und $R_6$ unabhängig voneinander für Wasserstoff, $C_1$-$C_6$-Alkyl, $C_1$-$C_6$-Alkoxycarbonyl stehen oder beide zusammen einen ankondensierten Phenylring bilden ;

n für eine der Zahlen 4 oder 5 steht ;

X Sauerstoff, Schwefel, $>C=O$ oder $>N-R_9$ bedeutet ;

$R_7$ und $R_8$ unabhängig voneinander für Wasserstoff, $C_1$-$C_6$-Alkyl oder $C_1$-$C_6$-Alkoxycarbonyl stehen ; und

$R_9$ Wasserstoff, $C_1$-$C_6$-Alkyl, Formyl; $C_1$-$C_6$-Alkanoyl oder $C_1$-$C_6$-Alkoxycarbonyl bedeutet, dadurch gekennzeichnet, dass man entweder

A) eine Verbindung der Formel II

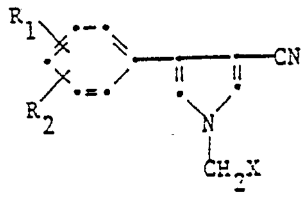

(II)

worin $R_1$ und $R_2$ die unter Formel I angegebenen Bedeutungen haben und X für OH oder Halogen steht, in Gegenwart einer Base und gegebenenfalls im Beisein eines Reaktionskatalysators bei Temperaturen von 0° bis 100 °C mit einer Verbindung der Formel III

$$H—Z \qquad (III)$$

umsetzt, worin Z die unter Formel I angegebenen Bedeutungen hat; oder

B) eine Verbindung der Formel IV

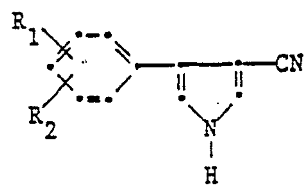

(IV)

worin $R_1$ und $R_2$ die unter Formel I angegebenen Bedeutungen haben, entweder in einem protischen Lösungsmittel bei Temperaturen von 0° bis 120 °C in Gegenwart eines basischen oder sauren Reaktionskatalysators mit Formaldehyde und einer Verbindung der Formel III umsetzt oder in einem aprotischen Lösungsmittel im Beisein eines basischen Reaktionskatalysators und bei Temperaturen von 0° bis 120 °C, vorzugsweise 20° bis 80 °C, mit 1,3,5-Trioxan oder Paraformaldehyd umsetzt.

2. Verfahren nach Anspruch 1, zur Herstellung von Verbindungen der Formel I, worin $R_1$ und $R_2$ unabhängig voneinander für Wasserstoff, Chlor, Methoxy oder Methylthio stehen; Z für eine der Gruppen

a) 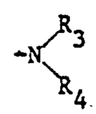 oder

b) 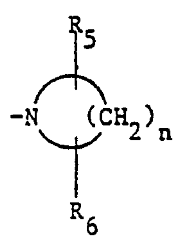 oder

c) 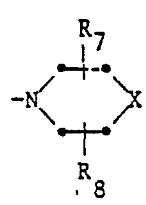

steht, wobei $R_3$ und $R_4$ unabhängig voneinander für Wasserstoff, $C_1$-$C_6$-Alkyl, durch Cyano, $C_1$-$C_3$-Alkoxy oder $C_1$-$C_3$-Alkoxycarbonyl substituiertes $C_1$-$C_3$-Alkyl, $C_3$-$C_4$-Alkenyl, $C_3$-$C_4$-Alkinyl, $C_5$-$C_6$-Cycloalkyl, unsubstituiertes oder durch Halogen, Methyl, Trifluormethyl und/oder Methoxy substituiertes Benzyl oder unsubstituiertes oder durch Halogen, Methyl, Trifluormethyl und/oder Methoxy substituiertes Phenyl stehen, mit der Massgabe, dass nur $R_3$ oder $R_4$ Wasserstoff sein kann; $R_5$ und $R_6$ unabhängig

voneinander für Wasserstoff, $C_1$-$C_3$-Alkyl oder $C_1$-$C_3$-Alkoxycarbonyl stehen oder beide zusammen einen ankondensierten Phenylring bilden ; n für eine der Zahlen 4 oder 5 steht ; X Sauerstoff, Schwefel, $>C{=}O$ oder $>N{-}R_9$ bedeutet ; $R_7$ und $R_8$ unabhängig voneinander für Wasserstoff, $C_1$-$C_3$-Alkyl oder $C_1$-$C_3$-Alkoxycarbonyl stehen ; und $R_9$ Wasserstoff, $C_1$-$C_3$-Alkyl, Formyl, $C_1$-$C_3$-Alkanoyl oder $C_1$-$C_3$-Alkoxycarbonyl bedeutet.

3. Verfahren nach Anspruch 2, zur Herstellung von Verbindungen der Formel I, worin $R_1$ in der 2-Position und $R_2$ in der 3-Position fixiert sind und unabhängig voneinander für Wasserstoff, Chlor, Methoxy oder Methylthio stehen ; Z für eine der Gruppen

a) 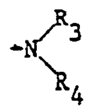 oder

b) 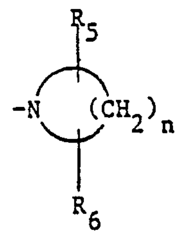 oder

c) 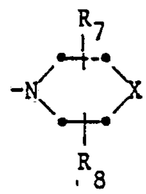

steht, wobei $R_3$ und $R_4$ unabhängig voneinander für $C_1$-$C_6$-Alkyl, durch Cyano, $C_1$-$C_3$-Alkoxy oder $C_1$-$C_3$-Alkoxycarbonyl substituiertes $C_1$-$C_2$-Alkyl, Allyl, Propargyl, Cyclopentyl, Cyclohexyl, Benzyl, Halobenzyl, Phenyl oder Halophenyl stehen ; $R_5$ und $R_6$ unabhängig voneinander für Wasserstoff, Methyl oder $C_1$-$C_3$-Alkoxycarbonyl stehen oder beide zusammen einen ankondensierten Phenylring bilden ; n für eine der Zahlen 4 oder 5 steht ; X Sauerstoff, Schwefel, $>C{=}O$ oder $>N{-}R_9$ bedeutet ; $R_7$ und $R_8$ unabhängig voneinander für Wasserstoff, Methyl oder $C_1$-$C_3$-Alkoxycarbonyl stehen ; und $R_9$ Wasserstoff, $C_1$-$C_2$-Alkyl, Formyl, $C_1$-$C_3$-Alkanoyl oder $C_1$-$C_3$-Alkoxycarbonyl bedeutet.

4. Verfahren nach Anspruch 3, zur Herstellung von Verbindungen der Formel I, worin $R_1$ für Wasserstoff oder 2-Chlor und $R_2$ für Wasserstoff oder 3-Chlor stehen ; Z für eine der Gruppen

a) 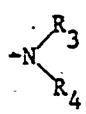 oder

b) 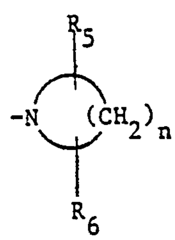 oder

c) 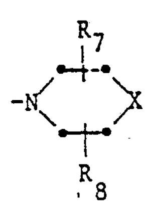

steht, wobei $R_3$ und $R_4$ unabhängig voneinander für $C_1$-$C_6$-Alkyl, durch Cyano, $C_1$-$C_3$-Alkoxy oder $C_1$-$C_3$-Alkoxycarbonyl substituiertes $C_1$-$C_2$-Alkyl, Allyl, Propargyl, Cyclopentyl, Cyclohexyl, Benzyl, Halobenzyl, Phenyl oder Halophenyl stehen ; $R_5$ und $R_6$ unabhängig voneinander für Wasserstoff, Methyl oder $C_1$-$C_3$-Alkoxycarbonyl stehen oder beide zusammen einen ankondensierten Phenylring bilden ; n für eine der Zahlen 4 oder 5 steht ; X Sauerstoff, Schwefel, $\geq$C=O oder >N—$R_9$ bedeutet ; $R_7$ und $R_8$ unabhängig voneinander für Wasserstoff, Methyl oder $C_1$-$C_3$-Alkoxycarbonyl stehen ; und $R_9$ Wasserstoff, $C_1$-$C_2$-Alkyl, Formyl, $C_1$-$C_3$-Alkanoyl oder $C_1$-$C_3$-Alkoxycarbonyl bedeutet.

5. Verfahren nach Anspruch 1, zur Herstellung einer Verbindung ausgewählt aus der Reihe
N-(N',N'-Dimethylaminomethyl)-3-(2,3-dichlorphenyl)-4-cyanopyrrol,
N-(N'-Benzyl-N'-methylaminomethyl)-3-(2,3-dichlorphenyl)-4-cyanopyrrol,
N-[N'-Methyl-N'-(2-cyanoethyl)-aminomethyl]-3-(2,3-dichlorphenyl)-4-cyanopyrrol,
N-[N'-Isopropyl-N'-(2-cyanoethyl)-aminomethyl]-3-(2,3-dichlorphenyl)-4-cyanopyrrol,
N-(Piperidin-1-ylmethyl)-3-(2,3-dichlorphenyl)-4-cyanopyrrol,
N-(3-Ethoxycarbonylpiperidin-1-ylmethyl)-3-(2-chlorphenyl)-4-cyanopyrrol,
N-(Morpholin-1-ylmethyl)-3-(2,3-dichlorphenyl)-4-cyanopyrrol,
N-(2,6-Dimethylmorpholin-1-ylmethyl)-3-(2-chlorphenyl)-4-cyanopyrrol,
N-[N'-Cyclopentyl-N'-(2-cyanoethyl)-aminomethyl]-3-(2,3-dichlorphenyl)-4-cyanopyrrol,
N-(2,6-Dimethylmorpholin-1-ylmethyl)-3-(2,3-dichlorphenyl)-4-cyanopyrrol,
N-(4-Acetylcarbonylpiperazin-1-ylmethyl)-3-(2,3-dichlorphenyl)-4-cyanopyrrol,
N-[N'-(2-Methoxyethyl)-N'-(2-cyanoethyl)-aminomethyl]-3-(2,3-dichlorphenyl)-4-cyanopyrrol,
N-[N'-Methyl-N'-(2-cyanoethyl)-aminomethyl]-3-(2-chlorphenyl)-4-cyanopyrrol,
N-[N'-Methyl-N'-(2-methoxyethyl)-aminomethyl]-3-(2,3-dichlorphenyl)-4-cyanopyrrol,
N-[N'-(n-Propyl)-N'-(2-cyanoethyl)-aminomethyl]-3-(2,3-dichlorphenyl)-4-cyanopyrrol,
N-[N'-(n-Butyl)-N'-(2-cyanoethyl)-aminomethyl]-3-(2,3-dichlorphenyl)-4-cyanopyrrol,
N-[N'-Methyl-N'-prop-2-enyl-aminomethyl]-3-(2,3-dichlorphenyl)-4-cyanopyrrol,
N-[N'-Propargyl-N'-cyclohexyl-aminomethyl]-3-(2,3-dichlorphenyl)-4-cyanopyrrol.

6. Verfahren nach Anspruch 1, dadurch gekennzeichnet, dass man als Base eine organische oder anorganische Base und als Säure eine organische oder eine Mineralsäure einsetzt.

7. Mikrobizides Mittel zur Bekämpfung oder Verhütung eines Befalls von lebenden Pflanzen und/oder zur Konservierung von verderblichem Lagergut pflanzlicher oder tierischer Herkunft, dadurch gekennzeichnet, dass es als mindestens eine aktive Komponente eine der in Anspruch 1 definierten Verbindungen enthält.

8. Mittel gemäss Anspruch 7, dadurch gekennzeichnet, dass es als aktive Komponente mindestens eine der in den Ansprüchen 2 bis 5 definierten Verbindungen enthält.

9. Mittel nach einem der Ansprüche 7 oder 8, dadurch gekennzeichnet, dass es 0,1 bis 99 % eines Wirkstoffs der Formel I, 99,9 bis 1 % eines festen oder flüssigen Zusatzstoffes und 0 bis 25 % eines Tensides enthält.

10. Mittel nach Anspruch 9, dadurch gekennzeichnet, dass es 0,1 bis 95 % eines Wirkstoffes der Formel I 99,8 bis 5 % eines festen oder flüssigen Zusatzstoffes und 0,1 bis 25 % eines Tensides enthält.

11. Verfahren zur Herstellung eines in den Ansprüchen 7 bis 10 definierten (agro)chemischen Mittels, dadurch gekennzeichnet, dass man mindestens eine Verbindung der Formel I gemäss einem der Ansprüche 1 bis 5 mit geeigneten festen oder flüssigen Zusatzstoffen und Tensiden innig vermischt.

12. Verfahren zur Bekämpfung oder Verhütung eines Befalls von Kulturpflanzen durch phytopathogene Mikroorganismen, dadurch gekennzeichnet, dass man eine Verbindung der Formel I gemäss einem der Ansprüche 1 bis 5 auf die Pflanze, Pflanzenteile oder deren Standord appliziert.

13. Verfahren zur Konservierung bzw. zum Schutz von Vorrats- oder Lagergut pflanzlicher und/oder tierischer Herkunft vor schädlichen Mikroorganismen durch Behandlung des Gutes mit mindestens einer mikrobizid wirksamen Menge einer Verbindung der Formel I gemäss einem der Ansprüche 1 bis 5.

14. Verwendung von Verbindungen der Formel I gemäss Anspruch 1 zur Bekämpfung und/oder zur präventiven Verhütung eines Befalls von Mikroorganismen.

15. Verwendung gemäss Anspruch 14, dadurch gekennzeichnet, dass es sich bei den Mikroorganismen und phytopathogene Pilze handelt.

**Claims** (for the Contracting States : GB, BE, DE, FR, IT, LU, NL, SE, CH, LI)

1. Compounds of the formula I

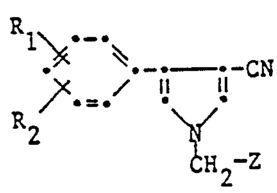

(I)

wherein

$R_1$ and $R_2$ are each independently hydrogen, halogen, methoxy or methylthio ;

Z is a group

a) 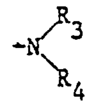 or

b) 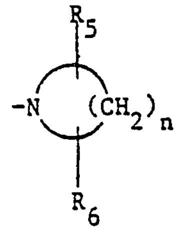 or

c) 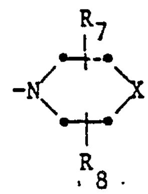

wherein

$R_3$ and $R_4$ are each independently hydrogen, $C_1$-$C_6$-alkyl which is unsubstituted or substituted by cyano, $C_1$-$C_6$-alkoxy or $C_1$-$C_6$-alkoxycarbonyl ; or $C_3$-$C_6$-alkenyl, $C_3$-$C_6$-alkynyl, $C_3$-$C_7$-cycloalkyl, benzyl or benzyl which is substituted by halogen, $C_1$-$C_6$-alkyl, $C_1$-$C_6$-haloalkyl and/or $C_1$-$C_6$-alkoxy, or phenyl or phenyl which is substituted by halogen, $C_1$-$C_6$-alkyl, $C_1$-$C_6$-haloalkyl and/or $C_1$-$C_6$-alkoxy, with the proviso that only $R_3$ or $R_4$ may be hydrogen ;

$R_5$ and $R_6$ are each independently hydrogen, $C_1$-$C_6$-alkyl, $C_1$-$C_6$-alkoxycarbonyl, or both taken together form a fused phenyl ring ;

n is 4 or 5 ;

X is oxygen, sulfur, $>C=O$ or $>N-R_9$ ;

$R_7$ and $R_8$ are each independently hydrogen, $C_1$-$C_6$-alkyl or $C_1$-$C_6$-alkoxycarbonyl ; and

$R_9$ is hydrogen, $C_1$-$C_6$-alkyl, formyl, $C_1$-$C_6$-alkanoyl or $C_1$-$C_6$-alkoxycarbonyl.

2. Compounds of the formula I according to claim 1, wherein $R_1$ and $R_2$ are each independently hydrogen, chlorine, methoxy or methylthio ; Z is a group

a) 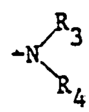 or

b) 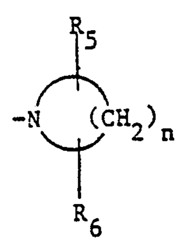 or

c) 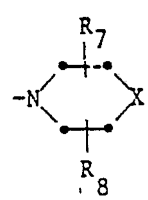

wherein $R_3$ and $R_4$ are each independently hydrogen, $C_1$-$C_6$-alkyl, $C_1$-$C_3$-alkyl which is substituted by cyano, $C_1$-$C_3$-alkoxy or $C_1$-$C_3$-alkoxycarbonyl ; or $C_3$-$C_4$-alkenyl, $C_3$-$C_4$-alkynyl, $C_5$-$C_6$-cycloalkyl, benzyl or benzyl which is substituted by halogen, methyl, trifluoromethyl and/or methoxy, or phenyl or phenyl which is substituted by halogen, methyl, trifluoromethyl and/or methoxy, with the proviso that only $R_3$ or $R_4$ may be hydrogen ; $R_5$ and $R_6$ are each independently hydrogen, $C_1$-$C_3$-alkyl or $C_1$-$C_3$-alkoxycarbonyl, or both taken together form a fused phenyl ring ; n is 4 or 5 ; X is oxygen, sulfur, $>C=O$ or $>N-R_9$ ; $R_7$ and $R_8$ are each independently hydrogen, $C_1$-$C_3$-alkyl or $C_1$-$C_3$-alkoxycarbonyl ; and $R_9$ is hydrogen, $C_1$-$C_3$-alkyl, formyl, $C_1$-$C_3$-alkanoyl or $C_1$-$C_3$-alkoxycarbonyl.

3. Compounds of the formula I according to claim 2, wherein $R_1$ is in the 2-position and $R_2$ is in the 3-position and each independently of the other is hydrogen, chlorine, methoxy or methylthio ; Z is a group

a) 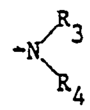 or

b) 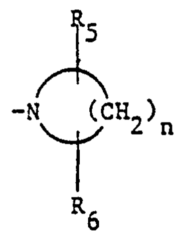 or

c) 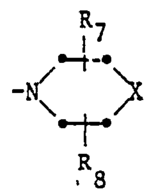

wherein $R_3$ and $R_4$ are each independently $C_1$-$C_6$-alkyl, $C_1$-$C_2$-alkyl which is substituted by cyano, $C_1$-$C_3$-alkoxy or $C_1$-$C_3$-alkoxycarbonyl ; or allyl, propargyl, cyclopentyl, cyclohexyl, benzyl, halobenzyl, phenyl or halophenyl ; $R_5$ and $R_6$ are each independently hydrogen, methyl or $C_1$-$C_3$-alkoxycarbonyl, or both taken together form a fused phenyl ring ; n is 4 or 5 ; X is oxygen, sulfur, $>C=O$ or $>N-R_9$ ; $R_7$ and $R_8$ are each independently hydrogen, methyl or $C_1$-$C_3$-alkoxycarbonyl ; and $R_9$ is hydrogen, $C_1$-$C_2$-alkyl, formyl, $C_1$-$C_3$-alkanoyl or $C_1$-$C_3$-alkoxycarbonyl.

4. Compounds of the formula I according to claim 3, wherein $R_1$ is hydrogen or 2-chloro and $R_2$ is hydrogen or 3-chloro ; Z is a group

a) 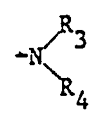 or

b) 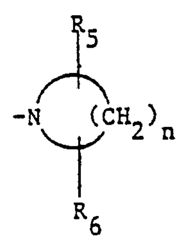 or

c) 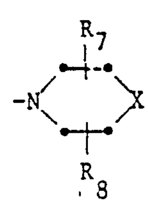

wherein $R_3$ and $R_4$ are each independently $C_1$-$C_6$-alkyl, $C_1$-$C_2$-alkyl which is substituted by cyano, $C_1$-$C_3$-alkoxy or $C_1$-$C_3$-alkoxycarbonyl ; or allyl, propargyl, cyclopentyl, cyclohexyl, benzyl, halobenzyl, phenyl or halophenyl ; $R_5$ and $R_6$ are each independently hydrogen, methyl or $C_1$-$C_3$-alkoxycarbonyl, or both taken together form a fused phenyl ring ; n is 4 or 5 ; X is oxygen or sulfur, $>C{=}O$ or $>N{-}R_9$ ; $R_7$ and $R_8$ are each independently hydrogen, methyl or $C_1$-$C_3$-alkoxycarbonyl ; and $R_9$ is hydrogen, $C_1$-$C_2$-alkyl, formyl, $C_1$-$C_3$-alkanoyl or $C_1$-$C_3$-alkoxycarbonyl.

5. A compound of the formula I according to claim 1, selected from the group consisting of
N-(N'-N'-dimethylaminomethyl)-3-(2,3-dichlorophenyl)-4-cyanopyrrole,
N-(N'-benzyl-N'-methylaminomethyl)-3-(2,3-dichlorophenyl)-4-cyanopyrrole,
N-[N'-methyl-N'-(2-cyanoethyl)-aminomethyl]-3-(2,3-dichlorophenyl)-4-cyanopyrrole,
N-[N'-isopropyl-N'-(2-cyanoethyl)-aminomethyl]-3-(2,3-dichlorophenyl)-4-cyanopyrrole,
N-(piperidin-1-ylmethyl)-3-(2,3-dichlorophenyl)-4-cyanopyrrole,
N-(3-ethoxycarbonylpiperidin-1-ylmethyl)-3-(2-chlorophenyl)-4-cyanopyrrole,
N-(morpholin-1-ylmethyl)-3-(2,3-dichlorophenyl)-4-cyanopyrrole,
N-(2,6-dimethylmorpholin-1-ylmethyl)-3-(2-chlorophenyl)-4-cyanopyrrole,
N-[N'-cyclopentyl-N'-(2-cyanoethyl)-aminomethyl]-3-(2,3-dichlorophenyl)-4-cyanopyrrole,
N-(2,6-dimethylmorpholin-1-ylmethyl)-3-(2,3-dichlorophenyl)-4-cyanopyrrole,
N-(4-acetylcarbonylpiperazin-1-ylmethyl)-3-(2,3-dichlorophenyl)-4-cyanopyrrole,
N-[N'-(2-methoxyethyl)-N'-(2-cyanoethyl)-aminomethyl]-3-(2,3-dichlorophenyl)-4-cyanopyrrole,
N-[N'-methyl-N'-(2-cyanoethyl)-aminomethyl]-3-(2-chlorophenyl)-4-cyanopyrrole,
N-[N'-methyl-N'-(2-methoxyethyl)-aminomethyl]-3-(2,3-dichlorophenyl)-4-cyanopyrrole,
N-[N'-(n-propyl)-N'-(2-cyanoethyl)-aminomethyl]-3-(2,3-dichlorophenyl)-4-cyanopyrrole,
N-[N'-(n-butyl)-N'-(2-cyanoethyl)-aminomethyl]-3-(2,3-dichlorophenyl)-4-cyanopyrrole,
N-[N'-methyl-N'-prop-2-enylaminomethyl]-3-(2,3-dichlorophenyl)-4-cyanopyrrole,
N-[N'-propargyl-N'-cyclohexylaminomethyl]-3-(2,3-dichlorophenyl)-4-cyanopyrrole.

6. A process for the preparation of the compounds of the formula I defined in claim 1, which comprises either

A) reacting a compound of the formula II

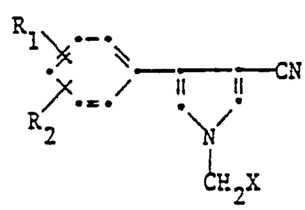

(II)

wherein $R_1$ and $R_2$ are as defined for formula I and X is OH or halogen, with a compound of the formula III

$$H{-}Z \qquad (III)$$

wherein Z is as defined for formula I, in the presence of a base and optionally of a catalyst, in the temperature range from 0° to 100 °C ; or

B) reacting a compound of the formula IV

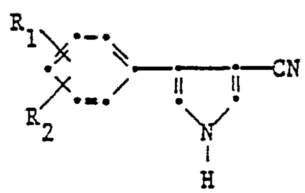

(IV)

wherein $R_2$ and $R_2$ are as defined for formula I, either with formaldehyde and a compound of the formula III, in a protic solvent in the temperature range from 0° to 120 °C, in the presence of a basic of acid catalyst, or with 1,3,5-trioxane or paraformaldehyde in an aprotic solvent in the presence of a basic catalyst and in the temperature range from 0° to 120 °C.

7. A microbicidal composition for controlling or preventing an attack on living plants and/or for preserving perishable storable goods of vegetable or animal origin, which composition contains as at least one active ingredient a compound as defined in claim 1.

8. A composition according to claim 7, which contains as active ingredient at least one compound as defined in claims 2 to 5.

9. A method of controlling or preventing an attack on cultivated plants by phytopathogenic microorganisms, which method comprises applying to said plants, to parts of the plants or to the locus thereof a compound of the formula I as defined in any one of claims 1 to 5.

10. A method of preserving storable goods of vegetable and/or animal origin or protecting such goods from harmful microorganisms by treating the goods with at least a microbicidally effective amount of a compound of the formula I according to any one of claims 1 to 5.

11. The use of compounds of the formula I according to claim 1 for controlling and/or preventing an attack by microorganisms.

12. The use according to claim 11 wherein the microorganisms are phytopathogenic fungi.

**Claims** (for the Contracting State AT)

1. A process for the preparation of a compound of the formula I

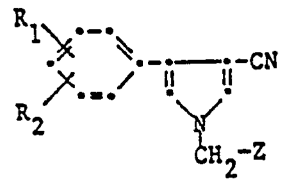

(I)

wherein

$R_1$ and $R_2$ are each independently hydrogen, halogen, methoxy or methylthio ;

Z is a group

a) 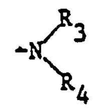 or

b) 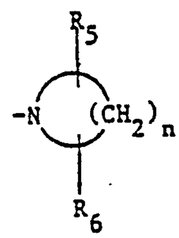 or

c) 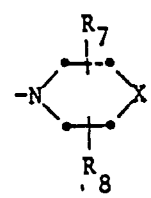

wherein

$R_3$ and $R_4$ are each independently hydrogen, $C_1$-$C_6$alkyl which is unsubstituted or substituted by cyano, $C_1$-$C_6$alkoxy or $C_1$-$C_6$alkoxycarbonyl ; or $C_3$-$C_6$alkenyl, $C_3$-$C_6$alkynyl, $C_3$-$C_7$cycloalkyl, benzyl or benzyl which is substituted by halogen, $C_1$-$C_6$alkyl, $C_1$-$C_6$haloalkyl and/or $C_1$-$C_6$alkoxy, or phenyl or phenyl which is substituted by halogen, $C_1$-$C_6$alkyl, $C_1$-$C_6$haloalkyl and/or $C_1$-$C_6$alkoxy, with the proviso that only $R_3$ or $R_4$ may be hydrogen ;

$R_5$ and $R_6$ are each independently hydrogen, $C_1$-$C_6$alkyl, $C_1$-$C_6$alkoxycarbonyl, or both taken together form a fused phenyl ring ;

n is 4 or 5 ;

X is oxygen, sulfur, $>C=O$ or $>N—R_9$ ;

$R_7$ and $R_8$ are each independently hydrogen, $C_1$-$C_6$alkyl or $C_{1-6}$alkoxycarbonyl ; and

$R_9$ is hydrogen, $C_1$-$C_6$alkyl, formyl, $C_1$-$C_6$alkanoyl or $C_1$-$C_6$alkoxycarbonyl, which process comprises either

A) reacting a compound of the formula II

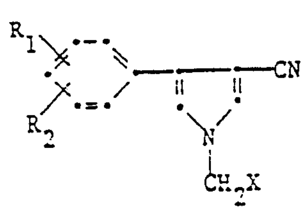

(II)

wherein $R_1$ and $R_2$ are as defined for formula I and X is OH or halogen, with a compound of the formula III

H—Z (III)

wherein Z is as defined for formula I, in the presence of a base and optionally of a catalyst, in the temperature range from 0° to 100 °C ; or

B) reacting a compound of the formula IV

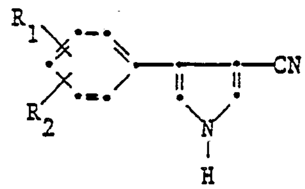 (IV)

wherein $R_1$ and $R_2$ are as defined for formula I, either with formaldehyde and a compound of the formula III, in a protic solvent in the temperature range from 0° to 120 °C, in the presence of a basic or acid catalyst, or with 1,3,5-trioxane or paraformaldehyde in an aprotic solvent in the presence of a basic catalyst and in the temperature range from 0° to 120 °C, preferably from 20° to 80 °C.

2. A process according to claim 1 for the preparation of compounds of the formula I wherein $R_1$ and $R_2$ are each independently hydrogen, chlorine, methoxy or methylthio ; Z is a group

a) 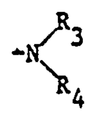 or

b) 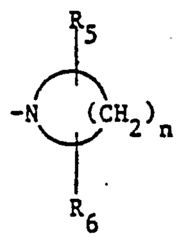 or

c) 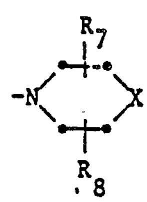

wherein $R_3$ and $R_4$ are each independently hydrogen, $C_1$-$C_6$alkyl, $C_1$-$C_3$alkyl which is substituted by cyano, $C_1$-$C_3$alkoxy or $C_1$-$C_3$alkoxycarbonyl ; or $C_3$-$C_4$alkenyl, $C_3$-$C_4$alkynyl, $C_5$-$C_6$cycloalky, benzyl or benzyl which is substituted by halogen, methyl trifluoromethyl and/or methoxy, or phenyl or phenyl which is substituted by halogen, methyl, trifluoromethyl and/or methoxy, with the proviso that only $R_3$ or $R_4$ may be hydrogen ; $R_5$ and $R_6$ are each independently hydrogen, $C_1$-$C_3$alkyl or $C_1$-$C_3$alkoxy-carbonyl, or both taken together form a fused phenyl ring ; n is 4 or 5 ; X is oxygen, sulfur, $>C=O$ or $>N—R_9$ ; $R_7$ and $R_8$ are each independently hydrogen, $C_1$-$C_3$alkyl or $C_1$-$C_3$alkoxycarbonyl ; and $R_9$ is hydrogen, $C_1$-$C_3$alkyl, formyl, $C_1$-$C_3$alkanoyl or $C_1$-$C_3$alkoxycarbonyl.

3. A process according to claim 2 for the preparation of compounds of the formula I wherein $R_1$ is in the 2-position and $R_2$ is in the 3-position and each independently of the other is hydrogen, chlorine, methoxy or methylthio ; Z is a group

a) 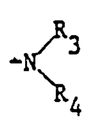 or

b) 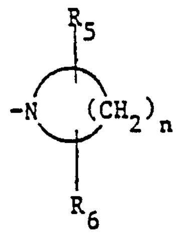 or

c) 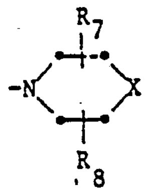

wherein $R_3$ and $R_4$ are each independently $C_1$-$C_6$alkyl, $C_1$-$C_2$alkyl which is substituted by cyano, $C_1$-$C_3$alkoxy or $C_1$-$C_3$alkoxycarbonyl ; or allyl, propargyl, cyclopentyl, cyclohexyl, benzyl, halobenzyl, phenyl or halophenyl ; $R_5$ and $R_6$ are each independently hydrogen, methyl or $C_1$-$C_3$alkoxycarbonyl, or both taken together form a fused phenyl ring ; n is 4 or 5 ; X is oxygen, sulfur, $>C=O$ or $>N—R_9$ ; $R_7$ and $R_8$ are each independently hydrogen, methyl or $C_1$-$C_3$alkoxycarbonyl ; and $R_9$ is hydrogen, $C_1$-$C_2$alkyl, formyl, $C_1$-$C_3$alkanoyl or $C_1$-$C_3$alkoxycarbonyl.

4. A process according to claim 3 for the preparation of compounds of the formula I wherein $R_1$ is hydrogen or 2-chloro and $R_2$ is hydrogen or 3-chloro ; Z is a group

a) 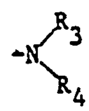 or

b) 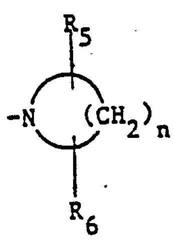 or

c) 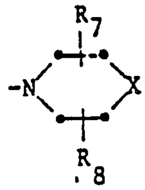

wherein $R_3$ and $R_4$ are each independently $C_1$-$C_6$alkyl, $C_1$-$C_2$alkyl which is substituted by cyano, $C_1$-$C_3$alkoxy or $C_1$-$C_3$alkoxycarbonyl ; or allyl, propargyl, cyclopentyl, cyclohexyl, benzyl, halobenzyl, phenyl or halophenyl ; $R_5$ and $R_6$ are each independently hydrogen, methyl or $C_1$-$C_3$alkoxycarbonyl, or both taken togeher form a fused phenyl ring ; n is 4 or 5 ; X is oxygen, sulfur, $>C=O$ or $>N—R_9$ ; $R_7$ and $R_8$ are each independently hydrogen, methyl or $C_1$-$C_3$alkoxycarbonyl ; and $R_9$ is hydrogen, $C_1$-$C_2$alkyl, formyl, $C_1$-$C_3$alkanoyl or $C_1$-$C_3$alkoxycarbonyl.

5. A process according to claim 1 for the preparation of a compound selected from the group consisting of

N-(N',N'-dimethylaminomethyl)-3-(2,3-dichlorophenyl)-4-cyanopyrrole,
N-(N'-benzyl-N'-methylaminomethyl)-3-(2,3-dichlorophenyl)-4-cyanopyrrole,
N-[N'-methyl-N'-(2-cyanoethyl)-aminomethyl]-3-(2,3-dichlorophenyl)-4-cyanopyrrole,
N-[N'-isopropyl-N'-(2-cyanoethyl)-aminomethyl]-3-(2,3-dichlorophenyl)-4-cyanopyrrole,
N-(piperidin-1-ylmethyl)-3-(2,3-dichlorophenyl)-4-cyanopyrrole,

N-(3-ethoxycarbonylpiperidin-1-ylmethyl)-3-(2-chlorophenyl)-4-cyanopyrrole,
N-(morpholin-1-ylmethyl)-3-(2,3-dichlorophenyl)-4-cyanopyrrole,
N-(2,6-dimethylmorpholin-1-ylmethyl)-3-(2-chlorophenyl)-4-cyanopyrrole,
N-[N'-cyclopentyl-N'-(2-cyanoethyl)-aminomethyl]-3-(2,3-dichlorophenyl)-4-cyanopyrrole,
N-(2,6-dimethylmorpholin-1-ylmethyl)-3-(2,3-dichlorophenyl)-4-cyanopyrrole,
N-(4-acetylcarbonylpiperazin-1-ylmethyl)-3-(2,3-dichlorophenyl)-4-cyanopyrrole,
N-[N'-(2-methoxyethyl)-N'-(2-cyanoethyl)-aminomethyl]-3-(2,3-dichlorophenyl)-4-cyanopyrrole,
N-[N'-methyl-N'-(2-cyanoethyl)-aminomethyl]-3-(2-chlorophenyl)-4-cyanopyrrole,
N-[N'-methyl-N'-(2-methoxyethyl)-aminomethyl]-3-(2,3-dichlorophenyl)-4-cyanopyrrole,
N-[N'-(n-propyl)-N'-(2-cyanoethyl)-aminomethyl]-3-(2,3-dichlorophenyl)-4-cyanopyrrole,
N-[N'-(n-butyl)-N'-(2-cyanoethyl)-aminomethyl]-3-(2,3-dichlorophenyl)-4-cyanopyrrole,
N-[N'-methyl-N'-prop-2-enylaminomethyl]-3-(2,3-dichlorophenyl)-4-cyanopyrrole,
N-[N'-propargyl-N'-cyclohexylaminomethyl]-3-(2,3-dichlorophenyl)-4-cyanopyrrole.

6. A process according to claim 1 wherein an organic or inorganic base is used as base and an organic or mineral acid is used as acid.

7. A microbicidal composition for controlling or preventing an attack on living plants and/or for preserving perishable storable goods of vegetable or animal origin, which composition contains as at least one active ingredient a compound as defined in claim 1.

8. A composition according to claim 7, which contains as active ingredient at least one compound as defined in claims 2 to 5.

9. A composition according to claim 7 or 8 which contains 0.1 to 99 % of a compound of the formula I, 99.9 to 1 % of a solid or liquid adjuvant and 0 to 25 % of a surfactant.

10. A composition according to claim 9 which contains 0.1 to 95 % of a compound of the formula I, 99.8 to 5 % of a solid or liquid adjuvant and 0.1 to 25 % of a surfactant.

11. A process for the preparation of an agro(chemical) composition as defined in claims 7 to 10, which process comprises intimately mixing at least one compound of the formula I according to any one of claims 1 to 5 with suitable solid or liquid adjuvants and surfactants.

12. A method of controlling or preventing an attack on cultivated plants by phytopathogenic microorganisms, which method comprises applying to said plants, to parts of the plants or to the locus thereof a compound of the formula I as defined in any one of claims 1 to 5.

13. A method of preserving storable goods of vegetable and/or animal origin or protecting such goods from harmful microorganisms by treating the goods with at least a microbicidally effective amount of a compound of the formula I according to any one of claims 1 to 5.

14. The use of compounds of the formula I according to claim 1 for controlling and/or preventing an attack by microorganisms.

15. The use according to claim 14 wherein the microorganisms are phytopathogenic fungi.

**Revendications** (pour les Etats contractants : GB, BE, DE, FR, IT, LU, NL, SE, CH, LI)

1. Composés de formule I

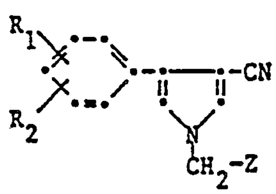

(I)

dans laquelle

$R_1$ et $R_2$ représentent chacun, indépendamment l'un de l'autre, l'hydrogène, un halogène, un groupe méthoxy ou méthylthio ;

Z représente l'un des groupes

a) 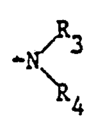 ou

b) 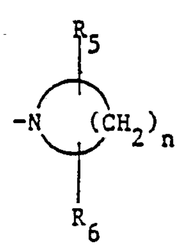 ou

c) 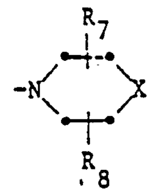

dans lesquels

$R_3$ et $R_4$ représentent chacun, indépendamment l'un de l'autre, l'hydrogène, un groupe alkyle en $C_1$-$C_6$ alcényle en $C_3$-$C_6$, alcynyle en $C_3$-$C_6$, cycloalkyle en $C_3$-$C_7$ non substitué ou substitué par des groupes cyano, alcoxy en $C_1$-$C_6$ ou (alcoxy en $C_1$-$C_6$)-carbonyle, un groupe benzyle non substitué ou substitué par des halogènes, des groupes alkyle en $C_1$-$C_6$, halogénoalkyle en $C_1$-$C_6$ et/ou alcoxy en $C_1$-$C_6$ ou un groupe phényle non substitué ou substitué par des halogènes, des groupes alkyle en $C_1$-$C_6$, halogénoalkyle en $C_1$-$C_6$ et/ou alcoxy en $C_1$-$C_6$, étant spécifié toutefois qu'un seul des symboles $R_3$ ou $R_4$ peut représenter l'hydrogène ;

$R_5$ et $R_6$ représentent chacun, indépendamment l'un de l'autre, l'hydrogène, un groupe alkyle en $C_1$-$C_6$, (alcoxy en $C_1$-$C_6$)-carbonyle ou bien forment ensemble un noyau phényle condensé ;

n est égal à 4 ou 5 ;

X représente l'oxygène, le soufre, un groupe $>C{=}O$ ou $>N{-}R_9$ ;

$R_7$ et $R_8$ représentent chacun, indépendamment l'un de l'autre, l'hydrogène, un groupe alkyle en $C_1$-$C_6$ ou (alcoxy en $C_1$-$C_6$)-carbonyle, et

$R_9$ représente l'hydrogène, un groupe alkyle en $C_1$-$C_6$, un groupe formyle ; un groupe alcanoyle en $C_1$-$C_6$ ou (alcoxy en $C_1$-$C_6$)-carbonyle.

2. Composés de formule I selon la revendication 1, dans lesquels $R_1$ et $R_2$ représentent chacun, indépendamment l'un de l'autre, l'hydrogène, le chlore, un groupe méthoxy ou méthylthio ; Z représente l'un des groupes

a) 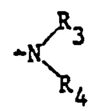 ou

b) 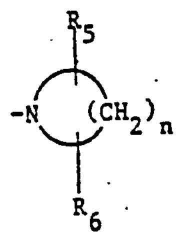 ou

c) 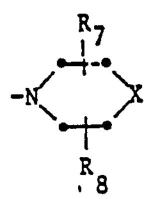

dans lesquels $R_3$ et $R_4$ représentent chacun, indépendamment l'un de l'autre, l'hydrogène, un groupe alkyle en $C_1$-$C_6$, un groupe alkyle en $C_1$-$C_3$, alcényle en $C_3$-$C_4$, alcynyle en $C_3$-$C_4$, cycloalkyle en $C_5$-$C_6$ substitué par des groupes cyano, alcoxy en $C_1$-$C_3$ ou (alcoxy en $C_1$-$C_3$)-carbonyle, un groupe benzyle non substitué ou substitué par des halogènes, des groupes méthyle, trifluorométhyle et/ou méthoxy, ou un groupe phényle non substitué ou substitué par des halogènes, des groupes méthyle, trifluorométhyle et/ou méthoxy, étant spécifié toutefois qu'un seul des symboles $R_3$ et $R_4$ peut représenter l'hydrogène ; $R_5$ et $R_6$ représentent chacun, indépendamment l'un de l'autre, l'hydrogène, un groupe alkyle en $C_1$-$C_3$ ou (alcoxy en $C_1$-$C_3$)-carbonyle ou bien forment ensemble un noyau phényle condensé ; n est égal à 4 ou 5 ; X représente l'oxygène, le soufre, un groupe $>C{=}O$ ou $>N{-}R_9$ ; $R_7$ et $R_8$ représentent chacun, indépendamment l'un de l'autre, l'hydrogène, un groupe alkyle en $C_1$-$C_3$ ou (alcoxy en $C_1$-$C_3$)-carbonyle ; et $R_9$ représente l'hydrogène, un groupe alkyle en $C_1$-$C_3$, formyle, alcanoyle en $C_1$-$C_3$ ou (alcoxy en $C_1$-$C_3$)-carbonyle.

3. Composés de formule I selon la revendication 2, dans lesquels $R_1$ est fixé en position 2 et $R_2$ en position 3 et ces deux symboles représentent chacun, indépendamment l'un de l'autre, l'hydrogène, le chlore, un groupe méthoxy ou méthylthio ; Z représente l'un des groupes

a) 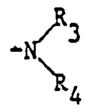 ou

b) 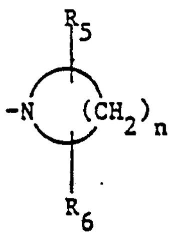 ou

c) 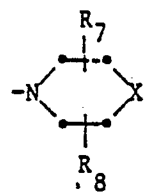

dans lesquels $R_3$ et $R_4$ représentent chacun, indépendamment l'un de l'autre, un groupe alkyle en $C_1$-$C_6$, un groupe alkyle en $C_1$-$C_2$, allyle, propargyle, cyclopentyle, cyclohexyle, benzyle, halogénobenzyle, phényle ou halogénophényle substitué par des groupes cyano, alcoxy en $C_1$-$C_3$ ou (alcoxy en $C_1$-$C_3$)-carbonyle ; $R_5$ et $R_6$ représentent chacun, indépendamment l'un de l'autre, l'hydrogène, un groupe méthyle ou (alcoxy en $C_1$-$C_3$)-carbonyle ou bien forment ensemble un noyau phényle condensé ; n est égal à 4 ou 5 ; X représente l'oxygène, le soufre, un groupe $>C{=}O$ ou $>N{-}R_9$ ; $R_7$ et $R_8$ représentent chacun, indépendamment l'un de l'autre, l'hydrogène, un groupe méthyle ou (alcoxy en $C_1$-$C_3$)-carbonyle ; et $R_9$ représente l'hydrogène, un groupe alkyle en $C_1$-$C_2$, formyle, alcanoyle en $C_1$-$C_3$ ou (alcoxy en $C_1$-$C_3$)-carbonyle.

4. Composés de formule I selon la revendication 3, dans lesquels $R_1$ représente l'hydrogène ou un substituant 2-chloro et $R_2$ représente l'hydrogène ou un substituant 3-chloro ; Z représente l'un des groupes

a) 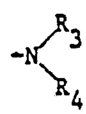 ou

b) 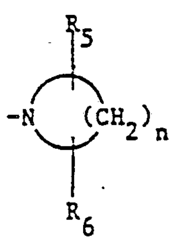 ou

c) 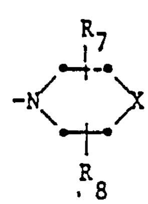

dans lesquels $R_3$ et $R_4$ représentent chacun, indépendamment l'un de l'autre, un groupe alkyle en $C_1$-$C_6$, un groupe alkyle en $C_1$-$C_2$, allyle, propargyle, cyclopentyle, cyclohexyle, benzyle, halogénobenzyle, phényle ou halogénophényle substitué par des groupes cyano, alcoxy en $C_1$-$C_3$ ou (alcoxy en $C_1$-$C_3$)-carbonyle ; $R_5$ et $R_6$ représentent chacun, indépendamment l'un de l'autre, l'hydrogène, un groupe méthyle ou (alcoxy en $C_1$-$C_3$)-carbonyle ou bien forment ensemble un noyau phényle condensé ; n est égal à 4 ou 5 ; X représente l'oxygène, le soufre, un groupe $>C{=}O$ ou $>N{-}R_9$ ; $R_7$ et $R_8$

représentent chacun, indépendamment l'un de l'autre, l'hydrogène, un groupe méthyle ou (alcoxy en $C_1$-$C_3$)-carbonyle ; et $R_9$ représente l'hydrogène, un groupe alkyle en $C_1$-$C_2$, formyle, alcanoyle en $C_1$-$C_3$ ou (alcoxy en $C_1$-$C_3$)-carbonyle.

5. Un composé de formule I selon la revendication 1, choisi dans le groupe suivant :
N-(N',N'-diméthylaminométhyl)-3-(2,3-dichlorophényl)-4-cyanopyrrole,
N-(N'-benzyl-N'-méthylaminométhyl)-3-(2,3-dichlorophényl)-4-cyanopyrrole,
N-[N'-méthyl-N'-(2-cyanéthyl)-aminométhyl]-3-(2,3-dichlorophényl)-4-cyanopyrrole,
N-[N'-isopropyl-N'-(2-cyanéthyl)-aminométhyl]-3-(2,3-dichlorophényl)-4-cyanopyrrole,
N-(pipéridine-1-ylméthyl)-3-(2,3-dichlorophényl)-4-cyanopyrrole,
N-(3-éthoxycarbonylpipéridine-1-ylméthyl)-3-(2-chlorophényl)-4-cyanopyrrole,
N-(morpholine-1-ylméthyl)-3-(2,3-dichlorophényl)-4-cyanopyrrole,
N-(2,6-diméthylmorpholine-1-ylméthyl)-3-(2-chlorophényl)-4-cyanopyrrole,
N-[N'-cyclopentyl-N'-(2-cyanéthyl)-aminométhyl]-3-(2,3-dichlorophényl)-4-cyanopyrrole,
N-(2,6-diméthylmorpholine-1-ylméthyl)-3-(2,3-dichlorophényl)-4-cyanopyrrole,
N-(4-acétylcarbonylpipérazine-1-ylméthyl)-3-(2,3-dichlorophényl)-4-cyanopyrrole,
N-[N'-(2-méthoxyéthyl)-N'-(2-cyanéthyl)-aminométhyl]-3-(2,3-dichlorophényl)-4-cyanopyrrole,
N-[N'-méthyl-N'-(2-cyanéthyl)-aminométhyl]-3-(2-chlorophényl)-4-cyanopyrrole,
N-[N'-méthyl-N'-(2-méthoxyéthyl)-aminométhyl]-3-(2,3-dichlorophényl)-4-cyanopyrrole,
N-[N'-(n-propyl)-N'-(2-cyanéthyl)-aminométhyl]-3-(2,3-dichlorophényl)-4-cyanopyrrole,
N-[N'-(n-butyl)-N'-(2-cyanéthyl)-aminométhyl]-3-(2,3-dichlorophényl)-4-cyanopyrrole,
N-[N'-méthyl-N'-propa-2-ényl-aminométhyl]-3-(2,3-dichlorophényl)-4-cyanopyrrole,
N-[N'-propargyl-N'-cyclohexyl-aminométhyl]-3-(2,3-dichlorophényl)-4-cyanopyrrole.

6. Procédé de préparation des composés de formule I définis dans la revendication 1, caractérisé en ce que :

A) on fait réagir un composé de formule II

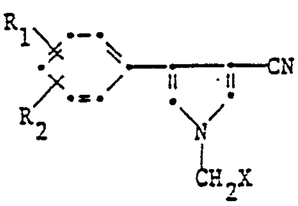

(II)

dans laquelle $R_1$ et $R_2$ ont les significations indiquées en référence à la formule I et X représente un groupe OH ou un halogène, en présence d'une base et, le cas échéant, en présence d'un catalyseur de réaction, à des températures de 0 à 100 °C, avec un composé de formule III

$$H—Z \quad (III)$$

dans laquelle Z a les significations indiquées en référence à la formule I ; ou bien

B) on fait réagir un composé de formule IV

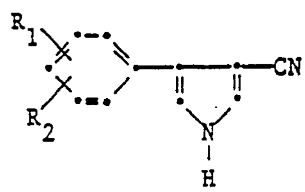

(IV)

dans laquelle $R_1$ et $R_2$ ont les significations indiquées en référence à la formule I, soit, dans un solvant protonique à des températures de 0 à 120 °C en présence d'un catalyseur de réaction basique ou acide, avec le formaldéhyde et un composé de formule III, soit dans un solvant aprotonique en présence d'un catalyseur de réaction basique et à des températures de 0 à 120 °C, avec le 1,3,5-trioxanne ou le paraformaldéhyde.

7. Produit microbicide pour combattre ou prévenir une attaque de plantes vivantes et/ou pour la conservation de marchandises périssables d'origine végétale ou animale, caractérisé en ce qu'il contient au moins un composant actif consistant en l'un des composés définis dans la revendication 1.

8. Produit selon la revendication 7, caractérisé en ce qu'il contient au moins un composant actif consistant en l'un des composés définis dans les revendications 2 à 5.

9. Procédé pour combattre ou prévenir une attaque de végétaux cultivés par des microorganismes phytopathogènes, caractérisé en ce que l'on applique à la plante, à des parties de la plante ou à son habitat un composé de formule I selon l'une des revendications 1 à 5.

10. Procédé pour conserver ou protéger des réserves ou marchandises d'origine végétale et/ou

animale contre des microorganismes nuisibles par traitement de la marchandise à l'aide d'au moins une quantité microbicide efficace d'un composé de formule I selon l'une des revendications 1 à 5.

11. Utilisation des composés de formule I selon la revendication 1 pour combattre et/ou prévenir une attaque par des microorganismes.

12. Utilisation selon la revendication 11, caractérisée en ce que les microorganismes sont des mycètes phytopathogènes.

**Revendications** (pour l'Etat contractant AT)

1. Procédé de préparation d'un composé de formule I

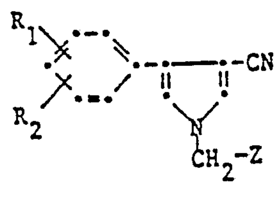

(I)

dans laquelle

$R_1$ et $R_2$ représentent chacun, indépendamment l'un de l'autre, l'hydrogène, un halogène, un groupe méthoxy ou méthylthio ;

Z représente l'un des groupes

a) 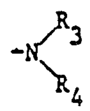 ou

b) 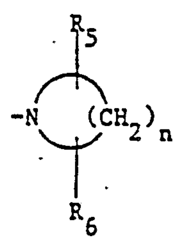 ou

c) 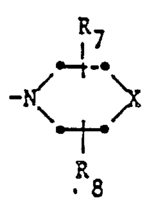

dans lesquels

$R_3$ et $R_4$ représentent chacun, indépendamment l'un de l'autre, l'hydrogène, un groupe alkyle en $C_1$-$C_6$, alcényle en $C_3$-$C_6$, alcynyle en $C_3$-$C_6$, cycloalkyle en $C_3$-$C_7$ non substitué ou substitué par des groupes cyano, alcoxy en $C_1$-$C_6$ ou (alcoxy en $C_1$-$C_6$)-carbonyle, un groupe benzyle non substitué ou substitué par des halogènes, des groupes alkyle en $C_1$-$C_6$, halogénoalkyle en $C_1$-$C_6$ et/ou alcoxy en $C_1$-$C_6$, ou un groupe phényle non substitué ou substitué par des halogènes, des groupes alkyle en $C_1$-$C_6$, halogénoalkyle en $C_1$-$C_6$ et/ou alcoxy en $C_1$-$C_6$, étant spécifié toutefois qu'un seul des symboles $R_3$ ou $R_4$ peut représenter l'hydrogène ;

$R_5$ et $R_6$ représentent chacun, indépendamment l'un de l'autre, l'hydrogène, un groupe alkyle en $C_1$-$C_6$, (alcoxy en $C_1$-$C_6$)-carbonyle ou forment ensemble un noyau phényle condensé ;

n est égal à 4 ou 5 ;

X représente l'oxygène, le soufre, un groupe $>C{=}O$ ou $>N{-}R_9$ ;

$R_7$ et $R_8$ représentent chacun, indépendamment l'un de l'autre, l'hydrogène, un groupe alkyle en $C_1$-$C_6$ ou (alcoxy en $C_1$-$C_6$)-carbonyle, et

$R_9$ représente l'hydrogène, un groupe alkyle en $C_1$-$C_6$, formyle ; un groupe alcanoyle en $C_1$-$C_6$ ou (alcoxy en $C_1$-$C_6$)-carbonyle, caractérisé en ce que :

A) on fait réagir un composé de formule II

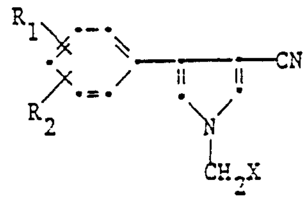

(II)

dans laquelle $R_1$ et $R_2$ ont les significations indiquées en référence à la formule I et X représente OH ou un halogène, en présence d'une base et, le cas échéant, en présence d'un catalyseur de réaction, à des températures de 0 à 100 °C, avec un composé de formule III

H—Z (III),

dans laquelle Z a les significations indiquées en référence à la formule I ; ou bien

B) on fait réagir un composé de formule IV

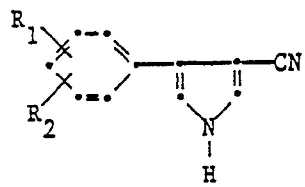

(IV)

dans laquelle $R_1$ et $R_2$ ont les significations indiquées en référence à la formule I, soit, dans un solvant protonique à des températures de 0 à 120 °C, en présence d'un catalyseur de réaction basique ou acide, avec le formaldéhyde et un composé de formule III, soit, dans un solvant aprotonique en présence d'un catalyseur de réaction basique et à des températures de 0 à 120 °C, de préférence de 20 à 80 °C, avec le 1,3,5-trioxanne ou le paraformaldéhyde.

2. Procédé selon la revendication 1, pour la préparation de composés de formule I dans laquelle $R_1$ et $R_2$ représentent chacun, indépendamment l'un de l'autre, l'hydrogène, le chlore, un groupe méthoxy ou méthylthio ; Z représente l'un des groupes

a) 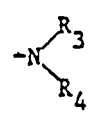 ou

b) 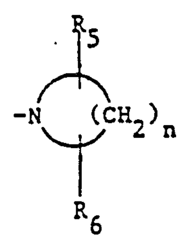 ou

c) 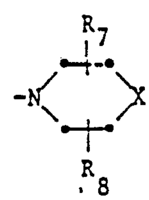

dans lesquels $R_3$ et $R_4$ représentent chacun, indépendamment l'un de l'autre, l'hydrogène, un groupe alkyle en $C_1$-$C_6$, un groupe alkyle en $C_1$-$C_3$, alcényle en $C_3$-$C_4$, alcynyle en $C_3$-$C_4$, cycloalkyle en $C_5$-$C_6$ substitué par des groupes cyano, alcoxy en $C_1$-$C_3$ ou (alcoxy en $C_1$-$C_3$)-carbonyle, un groupe benzyle non substitué ou substitué par des halogènes, des groupes méthyle, trifluorométhyle et/ou méthoxy ou un groupe phényle non substitué ou substitué par des halogènes, des groupes méthyle, trifluorométhyle et/ou méthoxy, étant spécifié toutefois qu'un seul des symboles $R_3$ ou $R_4$ peut représenter l'hydrogène ; $R_5$ et $R_6$ représentent chacun, indépendamment l'un de l'autre, l'hydrogène, un groupe alkyle en $C_1$-$C_3$

ou (alcoxy en $C_1$-$C_3$)-carbonyle ou forment ensemble un noyau phényle condensé ; n est égal à 4 ou 5 ; X représente l'oxygène, le soufre, un groupe $>C=O$ ou $>N-R_9$ ; $R_7$ et $R_8$ représentent chacun, indépendamment l'un de l'autre, l'hydrogène, un groupe alkyle en $C_1$-$C_3$ ou (alcoxy en $C_1$-$C_3$)-carbonyle ; et $R_9$ représente l'hydrogène, un groupe alkyle en $C_1$-$C_3$, formyle, alcanoyle en $C_1$-$C_3$ ou (alcoxy en $C_1$-$C_3$)-carbonyle.

3. Procédé selon la revendication 2, pour la préparation de composés de formule I dans laquelle $R_1$ est fixé en position 2 et $R_2$ en position 3, ces deux symboles représentant chacun, indépendamment l'un de l'autre, l'hydrogène, le chlore, un groupe méthoxy ou méthylthio ; Z représente l'un des groupes

a) 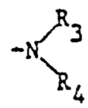 ou

b) 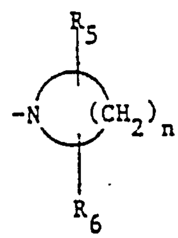 ou

c) 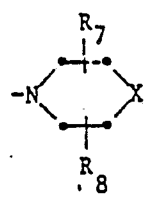

dans lesquels $R_3$ et $R_4$ représentent chacun, indépendamment l'un de l'autre, un groupe alkyle en $C_1$-$C_6$, un groupe alkyle en $C_1$-$C_2$, allyle, propargyle, cyclopentyle, cyclohexyle, benzyle, halogénobenzyle, phényle ou halogénophényle substitué par des groupes cyano, alcoxy en $C_1$-$C_3$ ou (alcoxy en $C_1$-$C_3$)-carbonyle ; $R_5$ et $R_6$ représentent chacun, indépendamment l'un de l'autre, l'hydrogène, un groupe méthyle ou (alcoxy en $C_1$-$C_3$)-carbonyle ou forment ensemble un noyau phényle condensé ; n est égal à 4 ou 5 ; X représente l'oxygène, le soufre, un groupe $>C=O$ ou $>N-R_9$ ; $R_7$ et $R_8$ représentent chacun, indépendamment l'un de l'autre, l'hydrogène, un groupe méthyle, ou (alcoxy en $C_1$-$C_3$)-carbonyle ; et $R_9$ représente l'hydrogène, un groupe alkyle en $C_1$-$C_2$, formyle, alcanoyle en $C_1$-$C_3$ ou (alcoxy en $C_1$-$C_3$)-carbonyle.

4. Procédé selon la revendication 3, pour la préparation de composés de formule I dans laquelle $R_1$ représente l'hydrogène ou un substituant 2-chloro et $R_2$ représente l'hydrogène ou un substituant 3-chloro ; Z représente l'un des groupes

a) 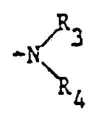 ou

b) 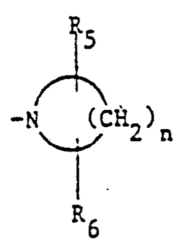 ou

c) 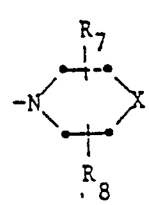

dans lesquels $R_3$ et $R_4$ représentent chacun, indépendamment l'un de l'autre, un groupe alkyle en $C_1$-$C_6$, un groupe alkyle en $C_1$-$C_2$, allyle, propargyle, cyclopentyle, cyclohexyle, benzyle, halogénobenzyle, phényle ou halogénophényle substitué par des groupes cyano, alcoxy en $C_1$-$C_3$ ou (alcoxy en $C_1$-$C_3$)-carbonyle ; $R_5$ et $R_6$ représentent chacun, indépendamment l'un de l'autre, l'hydrogène, un groupe méthyle ou (alcoxy en $C_1$-$C_3$)-carbonyle ou forment ensemble un noyau phényle condensé ; n est égal à 4 ou 5 ; X représente l'oxygène, le soufre, un groupe $>C=O$ ou $>N-R_9$ ; $R_7$ et $R_8$ représentent chacun, indépendamment l'un de l'autre, l'hydrogène, un groupe méthyle ou (alcoxy en $C_1$-$C_3$)-carbonyle ; et $R_9$ représente l'hydrogène, un groupe alkyle en $C_1$-$C_2$, formyle, alcanoyle en $C_1$-$C_3$ ou alcoxycarbonyle en $C_1$-$C_3$.

5. Procédé selon la revendication 1, pour la préparation d'un composé choisi dans le groupe suivant :

N-(N',N'-diméthylaminométhyl)-3-(2,3-dichlorophényl)-4-cyanopyrrole,
N-(N'-benzyl-N'-méthylaminométhyl)-3-(2,3-dichlorophényl)-4-cyanopyrrole,
N-[N'-méthyl-N'-(2-cyanéthyl)-aminométhyl]-3-(2,3-dichlorophényl)-4-cyanopyrrole,
N-[N'-isopropyl-N'-(2-cyanéthyl)-aminométhyl]-3-(2,3-dichlorophényl)-4-cyanopyrrole,
N-(pipéridine-1-ylméthyl)-3-(2,3-dichlorophényl)-4-cyanopyrrole,
N-(3-éthoxycarbonylpipéridine-1-ylméthyl)-3-(2-chlorophényl)-4-cyanopyrrole,
N-(morpholine-1-ylméthyl)-3-(2,3-dichlorophényl)-4-cyanopyrrole,
N-(2,6-diméthylmorpholine-1-ylméthyl)-3-(2-chlorophényl)-4-cyanopyrrole,
N-[N'-cyclopentyl-N'-(2-cyanéthyl)-aminométhyl]-3-(2,3-dichlorophényl)-4-cyanopyrrole,
N-(2,6-diméthylmorpholine-1-ylméthyl)-3-(2,3-dichlorophényl)-4-cyanopyrrole,
N-(4-acétylcarbonylpipérazine-1-ylméthyl)-3-(2,3-dichlorophényl)-4-cyanopyrrole,
N-[N'-(2-méthyoxyéthyl)-N'-(2-cyanéthyl)-aminométhyl]-3-(2,3-dichlorophényl)-4-cyanopyrrole,
N-[N'-méthyl-N'-(2-cyanéthyl)-aminométhyl]-3-(2-chlorophényl)-4-cyanopyrrole,
N-[N'-méthyl-N'-(2-méthoxyéthyl)-aminométhyl]-3-(2,3-dichlorophényl)-4-cyanopyrrole,
N-[N'-(n-propyl)-N'-(2-cyanéthyl)-aminométhyl]-3-(2,3-dichlorophényl)-4-cyanopyrrole,
N-[N'-(n-butyl)-N'-(2-cyanéthyl)-aminométhyl]-3-(2,3-dichlorophényl)-4-cyanopyrrole,
N-[N'-méthyl-N'-propa-2-ényl-aminométhyl]-3-(2,3-dichlorophényl)-4-cyanopyrrole,
N-[N'-propargyl-N'-cyclohexyl-aminométhyl]-3-(2,3-dichlorophényl)-4-cyanopyrrole.

6. Procédé selon la revendication 1, caractérisé en ce que l'on utilise en tant que base une base organique ou minérale et en tant qu'acide organique ou minéral.

7. Produit microbicide pour combattre ou prévenir une attaque de plantes vivantes et/ou pour conserver des marchandises périssables d'origine végétale ou animale, caractérisé en ce qu'il contient au moins un composant actif consistant en l'un des composés définis dans la revendication 1.

8. Produit selon la revendication 7, caractérisé en ce qu'il contient au moins un composant actif consistant en l'un des composés définis dans les revendications 2 à 5.

9. Produit selon l'une des revendications 7 ou 8, caractérisé en ce qu'il contient de 0,1 à 99 % d'une substance active de formule I, de 99,9 à 1 % d'un additif solide ou liquide et de 0 à 25 % d'un agent tensioactif.

10. Produit selon la revendication 9, caractérisé en ce qu'il contient de 0,1 à 95 % d'une substance active de formule I, de 99,8 à 5 % d'un additif solide ou liquide et de 0,1 à 25 % d'un agent tensioactif.

11. Procédé de préparation d'un produit (agro)- chimique défini dans les revendications 7 à 10, caractérisé en ce que l'on mélange intimement au moins un composé de formule I selon l'une des revendications 1 à 5 avec des additifs solides ou liquides et des agents tensioactifs appropriés.

12. Procédé pour combattre ou prévenir une attaque de végétaux cultivés par des microorganismes phytopathogènes, caractérisé en ce que l'on applique sur la plante, des parties de la plante ou son habitat un composé de formule I selon l'une des revendications 1 à 5.

13. Procédé pour conserver ou protéger des réserves ou marchandises d'origine végétale et/ou animale contre les microorganismes nuisibles par traitement de la marchandise à l'aide d'au moins une quantité microbicide efficace d'un composé de formule I selon l'une des revendications 1 à 5.

14. Utilisation de composés de formule I selon la revendication 1 pour combattre et/ou prévenir une attaque par des microorganismes.

15. Utilisation selon la revendication 14, caractérisée en ce que les microorganismes sont des mycètes phytopathogènes.